# EUROPEAN PATENT SPECIFICATION

(11) **EP 4 432 952 B1**
(45) Date of publication and mention of the grant of the patent: **14.05.2025**
(21) Application number: 22818723.3
(22) Date of filing: 18.11.2022
(51) Int. Cl.: A61B 34/10, A61B 34/20, A61B 34/00, A61B 34/32, A61B 5/00, G06T 7/00

(54) **CONTROL OF ROBOTIC ENDOVASCULAR DEVICES WITH FLUOROSCOPIC FEEDBACK**
STEUERUNG VON ROBOTISCHEN ENDOVASKULÄREN VORRICHTUNGEN MIT FLUOROSKOPISCHEM FEEDBACK
COMMANDE DE DISPOSITIFS ENDOVASCULAIRES ROBOTIQUES AVEC RÉTROACTION FLUOROSCOPIQUE

(30) Priority: 19.11.2021 US 202163281207 P; 02.02.2022 EP 22154694
(43) Date of publication of application: 25.09.2024
(73) Proprietor: Koninklijke Philips N.V., 5656 AG Eindhoven (NL)
(72) Inventor: BALICKI, Marcin A., 5656 AG Eindhoven (NL); FLEXMAN, Molly Lara, 5656 AG Eindhoven (NL)
(74) Representative: Philips Intellectual Property & Standards
(86) International application number: PCT/EP2022/082445
(87) International publication number: WO 2023/089117

(56) References cited:
- WO-A1-2010/025336
- US-A1- 2017 151 027
- US-A1- 2020 046 434
- US-A1- 2021 093 406

## Description

### BACKGROUND

Vascular passages are defined by delicate vascular walls. Endovascular devices are passed through vascular passages in endovascular interventional medical procedures, and constantly present risk of perforating the delicate vascular wall. Examples of endovascular devices include guidewires and catheters. In some endovascular interventional medical procedures, endovascular devices are navigated to treatment sites. A guidewire may be inserted endovascularly before a coaxial catheter is endovascularly guided along the guidewire to the treatment site. Medical imaging such as 2D perspective fluoroscopy may be used to provide image feedback to a skilled professional to assist in guiding the endovascular devices.

A typical endovascular interventional medical procedure using endovascular devices involves a set of discrete maneuvers. Each discrete maneuver may involve coordinating the motion of two endovascular devices to first deposit the guidewire in the target vascular branch, and then to advance the catheter over the guidewire.

Precise manipulation of multiple coaxial endovascular devices such as guidewire/catheter combinations requires superb image interpretation ability and hand-eye coordination, both of which are typically the product of years of experience. Experienced professionals obtain skills through observation and trial and error over many years. However, endovascular navigation is not routine for novices, and presents difficulties even for experienced professionals in cases that are considered difficult due, for example, to tortuous anatomy. Skilled professionals are sometimes unavailable for emergencies such as stroke treatments, and the absence of required skills presents heightened risks of perforating the delicate vascular walls, which may sometimes prove fatal. Prior art is found in US 2017/151027, US 2021/093406, WO 2010/025336 and US 2020/046434.

### SUMMARY

The invention is defined by independent claim 1.

According to a more detailed aspect of the present disclosure, a robotic device is configured to operate an interventional device for insertion into an anatomical structure of a subject. The interventional device comprises an outer device and an inner device movably positioned with the outer device. A method for controlling the robotic device includes receiving, from an imaging device, image data from an image of a portion of the interventional device and a branched intersection of a plurality of branches of the anatomical structure. The plurality of branches includes a main branch and a target branch which is branched from the main branch. The method also includes analyzing the image data to measure at least one of a location or an orientation of a distal portion of the outer device and of a distal portion of the inner device in the image, with respect to vasculature including the main branch and the target branch and an intersection of the main branch and the target branch in the image. The method further includes determining, based on analyzing the image data, a path for the interventional device through the anatomical structure using a plurality of modules of predefined motions stored in a memory; and controlling, based on determining the path for the interventional device, the robotic device to operate the interventional device through the path by a sequence of the predefined motions. The method further includes displaying the path on a display.

According to another aspect of the present disclosure, a robotic device is configured to operate an interventional device for insertion into an anatomical structure of a subject. The interventional device comprises an outer device and an inner device movably positioned within the outer device. A system for controlling the robotic device incudes an imaging interface, a user interface, a robotic device, a robotic device controller and a display. The imaging interface is configured to receive image data corresponding to an image from an imaging device. The image includes a portion of the interventional device and a branched intersection of a plurality of branches of the anatomical structure. The plurality of branches includes a main branch and a target branch which is branched from the main branch. The user interface is configured to receive input from a user. The robotic device is connected to the interventional device for operating the interventional device for insertion into the anatomical structure of the subject. The robotic device controller comprises a memory that stores instructions and a plurality of modules of predefined motions, and a processor that executes the instructions. When the instructions are executed by the processor, the robotic device controller is configured to analyze the image data to measure at least one of a location or an orientation of a distal portion of the outer device and of a distal portion of the inner device in the image, with respect to vasculature including the main branch and the target branch and an intersection of the main branch and the target branch in the image. The robotic device controller is also configured to determine, based on analyzing the image data, a path for the interventional device through the anatomical structure using modules of predefined motions stored in the memory. The robotic device controller is also configured to control the robotic device in response to the input from the user interface and based on determining the path for the interventional device, to operate the interventional device through the path by a sequence of the predefined motions. The display is configured to display at least the branch intersection of the plurality of branches of the anatomical structure, the path, the distal portion of the outer device and the distal portion of the inner device, while the robotic device controller controls the robotic device.

According to another aspect of the present disclosure, a robotic device is configured to operate an interventional device for insertion into an anatomical structure of a subject. The interventional device comprises an outer device and an inner device movably positioned within the outer device. A controller for controlling the robotic device includes a memory that stores instructions and a library of a plurality of modules of predefined motions for navigating through anatomical structures, and a processor that executes the instructions. When executed by the processor, the instructions cause the controller to receive, from an imaging device, image data from an image of a portion of the interventional device and a branched intersection of a plurality of branches of the anatomical structure. The plurality of branches includes a main branch and a target branch which is branched from the main branch. The instructions also cause the controller to analyze the image data to measure at least one of a location or an orientation of a distal portion of the outer device and of a distal portion of the inner device in the image, with respect to vasculature including the main branch and the target branch and an intersection of the main branch and the target branch in the image. The instructions also cause the controller to determine, based on analyzing the image data, a path for the interventional device through the anatomical structure using a plurality of modules of predefined motions stored in the memory. The instructions further cause the controller to control, based on a plurality of the predefined motions, the robotic device to operate the interventional device through the path by a sequence of the predefined motions. The instructions also cause the controller to display the path on a display.

In particular, an embodiment provides that the instructions further cause the controller to display a representation of different predefined motions as a part or the entirety of the said sequence. A non-claimed method further comprises displaying at least a portion of the path on a display (180) such that the predefined motions, as a part or the entirety of the said sequence, are represented.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A illustrates a system for control of robotic endovascular devices with fluoroscopic feedback, in accordance with a representative embodiment.
FIG. 1B illustrates a controller for control of robotic endovascular devices with fluoroscopic feedback, in accordance with a representative embodiment.
FIG. 1C illustrates another system for control of robotic endovascular devices with fluoroscopic feedback, in accordance with a representative embodiment.
FIG. 2A illustrates a method for control of robotic endovascular devices with fluoroscopic feedback, in accordance with a representative embodiment.
FIG. 2B illustrates another method for control of robotic endovascular devices with fluoroscopic feedback, in accordance with a representative embodiment.
FIG. 2C illustrates another method for control of robotic endovascular devices with fluoroscopic feedback, in accordance with a representative embodiment.
FIG. 2D illustrates another method for control of robotic endovascular devices with fluoroscopic feedback, in accordance with a representative embodiment.
FIG. 3 illustrates motions of an endovascular guidewire and catheter for control of robotic endovascular devices with fluoroscopic feedback, in accordance with a representative embodiment.
FIG. 4 illustrates alignment of an endovascular guidewire and catheter for control of robotic endovascular devices with fluoroscopic feedback, in accordance with a representative embodiment.
FIG. 5 illustrates a method for control of robotic endovascular devices with fluoroscopic feedback, in accordance with a representative embodiment.
FIG. 6A illustrates a graphical user interface for control of robotic endovascular devices with fluoroscopic feedback, in accordance with a representative embodiment.
FIG. 6B illustrates a method for control of robotic endovascular devices with fluoroscopic feedback, in accordance with the graphical user interface in FIG. 6A.
FIG. 7A illustrates a method for control of robotic endovascular devices with fluoroscopic feedback, in accordance with a representative embodiment.
FIG. 7B illustrates a graphical user interface for control of robotic endovascular devices with fluoroscopic feedback, in accordance with the method in FIG. 7A.
FIG. 8 illustrates a user interface for control of robotic endovascular devices with fluoroscopic feedback, in accordance with a representative embodiment.
FIG. 9A illustrates a user interface for control of robotic endovascular devices with fluoroscopic feedback, in accordance with a representative embodiment.
FIG. 9B illustrates a sequence of motions with an option to change or accept, for control of robotic endovascular devices with fluoroscopic feedback, in accordance with the user interface in FIG. 9A.
FIG. 9C illustrates another user interface for control of robotic endovascular devices with fluoroscopic feedback, in accordance with a representative embodiment.
FIG. 9D illustrates a sequence of motions with an option to change or accept, for control of robotic endovascular devices with fluoroscopic feedback, in accordance with the user interface in FIG. 9C.
FIG. 10 illustrates a computer system, on which a method for control of robotic endovascular devices with fluoroscopic feedback is implemented, in accordance with another representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Methods described hereafter do not form part of the claimed invention as defined by the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

As described herein, fluoroscopic imaging may be synchronized with servo control of endovascular navigation devices to guide the endovascular devices to anatomical targets.

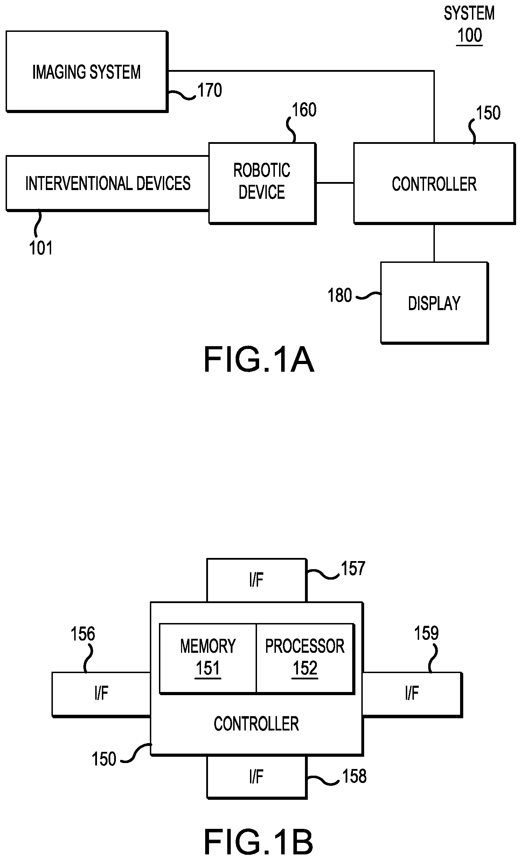

FIG. 1A illustrates a system 100 for control of robotic endovascular devices with fluoroscopic feedback, in accordance with a representative embodiment.

The system 100 in FIG. 1A is a system for control of robotic endovascular devices with fluoroscopic feedback and includes components that may be provided together or that may be distributed. The system 100 includes a controller 150, a robotic device 160, an imaging system 170 and a display 180. The robotic device 160 is configured to drive interventional devices 101 under the control of the controller 150. The interventional devices 101 are representative of a first interventional device and a second interventional device which is coaxial with the first interventional device. An example of the interventional devices 101 is an inner device and an outer device, such as a guidewire sliding inside a catheter. The interventional devices 101 are driven by the robotic device 160 under the control of the controller 150 and based on branched anatomical images where the interventional devices 101 have been identified.

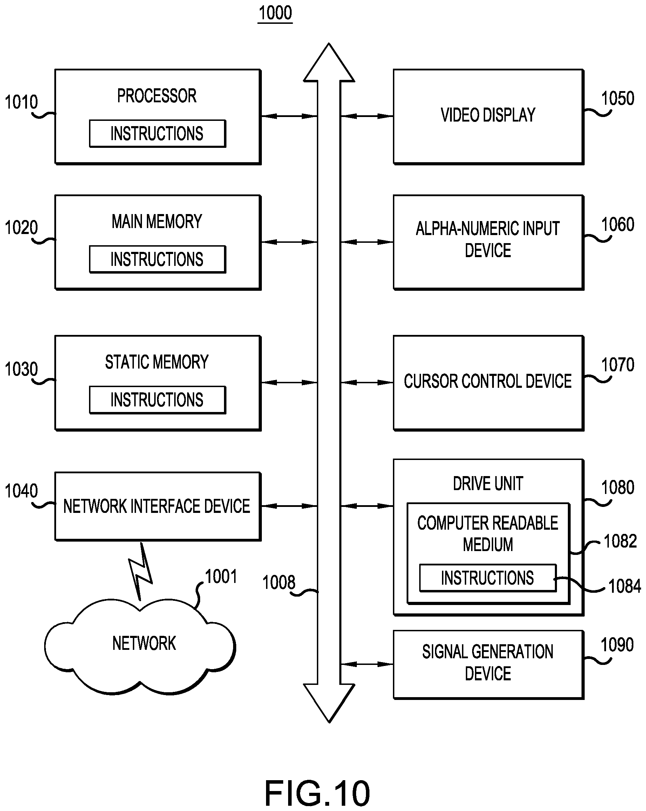

The controller 150 is further depicted in FIG. 1B, and includes at least a memory 151 that stores instructions and a processor 152 that executes the instructions. A computer that can be used to implement the controller 150 is depicted in FIG. 10, though a controller 150 may include more or fewer elements than depicted in FIG. 1B or in FIG. 10.

The controller 150 is configured to analyze images from the imaging system 170 and parametrize features of the interventional devices 101 in the images from the imaging system 170. For example, the controller 150 may use artificial intelligence (AI) to segment the interventional devices 101 in images. The controller 150 is also configured to detect operator input for a control mode. The controller 150 may create one or more goal reference metric(s) relative to one of the parameterized features of the interventional devices 101, or different goal reference metric(s) for multiple of the parameterized features of the interventional devices 101. The controller 150 may servo-drive the robotic device 160 so that a metric of a parameterized feature is minimized (or maximized) in the next image, or at least reduced (or increased) in the next image. The controller 150 may stop driving the robotic device 160 when the metric is within a tolerance value or the operator deactivates control. An example of a metric is 5 pixels, such as when a fluoroscopic image shows 5 pixels or more of the exposed inner device (the guidewire) protruding beyond the tip of the outer device (the catheter).

The robotic device 160 is controlled by the controller 150 to drive the interventional devices 101. The robotic device 160 is configured to drive the interventional devices in one or more degrees of freedom, such as in three dimensions and about one or more axes. The robotic device 160 may include a servo motor used to drive the interventional devices 101 under the control of the controller 150, and based on fluoroscopic feedback from the imaging system 170. The robotic device 160 may control one or more degrees of freedom of control for one or both of the interventional devices 101. The robotic device is controlled by the controller 150, so as to drive the motions of one or both of the interventional devices 101.

The imaging system 170 may be a fluoroscopic imaging system that captures fluoroscopic images of anatomy of a subject and the interventional devices 101 as the interventional devices 101 is inserted into the anatomy of the subject. The imaging system 170 may image the patient and the interventional devices 101 and may be movable directly by a user or under the control of the user. The imaging system 170 may be an interventional X-ray imaging system. An interventional X-ray imaging system may include an X-ray tube adapted to generate X-rays and an X-ray detector configured to acquire time-series X-ray images such as fluoroscopy images. Examples of such X-ray imaging systems include digital radiography-fluoroscopy systems such as ProxiDiagnost from Philips, fixed C-arm X-ray systems such as Azurion from Philips, and mobile C-arm X-ray systems such as Veradius from Philips.

The display 180 may be local to the controller 150 or may be remotely connected to the controller 150 via a standard web interface. The display 180 may be connected to the controller 150 via a local wired interface such as an Ethernet cable or via a local wireless interface such as a Wi-Fi connection. The display 180 is configured to display imaging content from the fluoroscopic images from the imaging system 170, along with supplementary depictions of the interventional devices 101 in the fluoroscopic images and one or more predefined motions used to generate a path through branches in the anatomy of the subject. In some embodiments, predefined motions used to generate a path through branches in the anatomy of the subject are not necessarily displayed, and the robotic device 160 controls the interventional devices 101 based on standing instructions. The display 180 may also be configured to display data and visual representations of a target anatomical structure, and data and visual representations of the interventional devices 101 relative to the target anatomical structure. The display 180 may be interfaced with other user input devices by which users can input instructions, including mouses, keyboards, thumbwheels and so on.

In some embodiments, the display 180 may display motions for the interventional device 101 to travel along a suggested path to a target vessel. The path through an anatomical structure using a sequence of the motions being displayed on the display 180 may be highlighted for confirmation by a user, so that the user may confirm the suggested path and the robotic device 160 may control the interventional device 101.

The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect other elements or components to the controller 150, as well as an interactive touch screen configured to display prompts to users and collect touch input from users.

FIG. 1B illustrates controller 150 for control of robotic endovascular devices with fluoroscopic feedback, in accordance with a representative embodiment.

The controller 150 includes a memory 151, a processor 152, a first interface 156, a second interface 157, a third interface 158, and a fourth interface 159. The memory 151 stores instructions which are executed by the processor 152. The memory 151 also stores a library of controlling tools related to specific motions of the interventional devices 101. The controlling tools in the library stored in the memory 151 may comprise instructions for alignment, retraction, rotation, advancement or projections based on expected motions/maneuvers. The processor 152 executes the instructions. The processor 152 may execute instructions to measure distances and/or orientations of the interventional devices 101 in the images and to parametrize the features of the interventional devices 101 in images. The analysis and parameterization by the processor may be performed based on the branched anatomy surrounding the interventional devices 101 in the images, along with predefined target in the anatomy in the images such as a target branch or intersection of branches in the images. The processor 152 may also execute instructions comprising direct encoder information from the robotic device 160, or from the interventional devices 101 such as from shape sensing sensors/devices which sense the shape of the interventional devices 101.

The interfaces of the controller include a first interface 156 to the robotic device 160, a second interface 157 to the imaging system 170, a third interface 158 to the display 180, and a fourth interface 159 to a user. The first interface 156, the second interface 157 and the third interface 158 may include ports, disk drives, wireless antennas, or other types of receiver circuitry. The first interface 156 may be a data interface that received data from the robotic device 160 and that provides instructions to the robotic device 160. The second interface 157 may be an image interface that receives data of images and of the identified interventional devices 101 in the images from the imaging system 170. The third interface 158 may be a data interface and an image interface that provides data and images to the display 180. The fourth interface 159 may include one or more user interfaces, such as a mouse, a keyboard, a microphone, a video camera, a gesture tracking system, a touchscreen display, or other forms of interactive user interfaces. The fourth interface 159 is therefore a user interface that receives user inputs, including inputs to set an operation mode for the robotic device 160 and inputs to make selections such as a selection of a predefined motion among multiple selectable options provided on the display 180.

The controller 150 is configured to control the interventional devices 101 using fluoroscopic feedback from images from the imaging system 170. The controller 150 may be provided as a stand-alone component as shown in FIG. 1B, or as a component of a device such as a workstation which also includes the display 180 in FIG. 1A. The controller 150 may perform some of the operations described herein directly and may implement other operations described herein indirectly. For example, the controller 150 may directly analyze fluoroscopic images from the imaging system 170 and may directly control the robotic device 160 to drive the interventional devices 101. The controller 150 may indirectly control other operations such as by generating and transmitting content to be displayed on the display 180, or commands to the robotic device 160. Accordingly, the processes implemented by the controller 150 when the processor 152 executes instructions from the memory 151 may include steps not directly performed by the controller 150.

When the processor 152 executes the instructions in the memory 151, the controller 150 is configured to perform a variety of processes corresponding to specific predetermined motions from a library of predetermined motions stored in the memory 151.

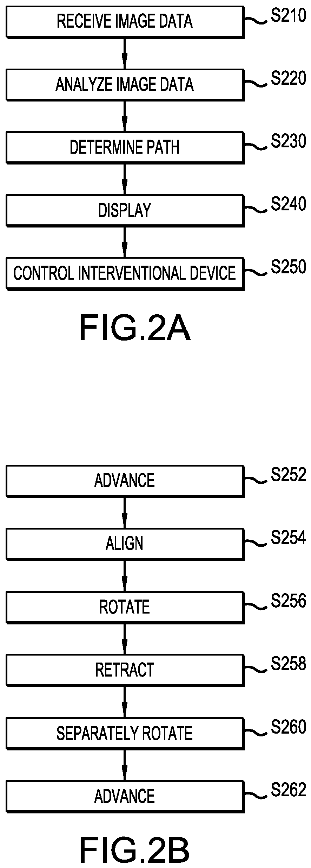

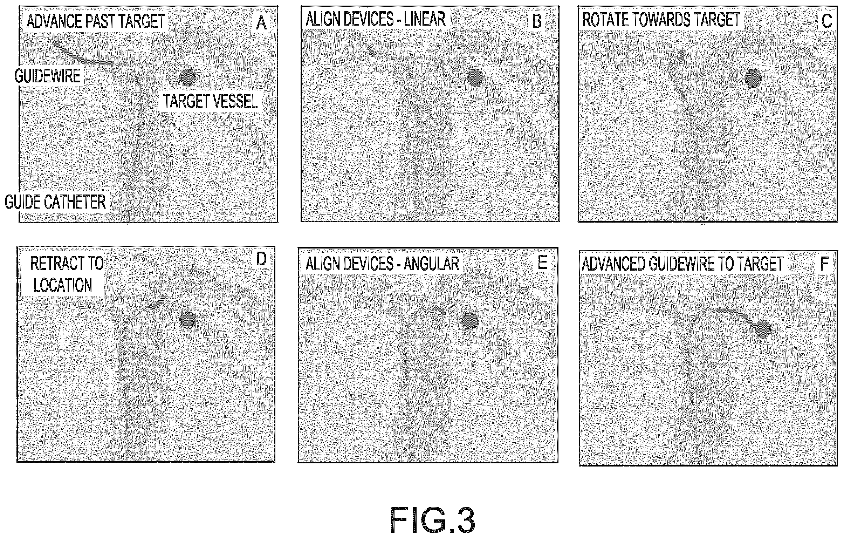

As an initial example, the controller 150 may control the robotic device 160 to advance either or both of the interventional devices 101 to a location. Advancing is technically more complex than retraction due to the potential of interacting with tissue such as the vascular walls. Advancing under the control of the robotic device 160 may be limited to the entrance of a branch, or may be more tolerated such as when advancing inside a main branch rather than a smaller-diameter target branch which is branched from the main branch. Advancing past a target is explained further with respect to S 252 in FIG. 2B and with respect to A in FIG. 3. In some embodiments, either or both of the interventional devices 101 may be advanced to just before the branched intersection, such as when the interventional devices 101 are shaped to point up towards an intersection instead of down towards the intersection as in most embodiments described herein.

As another example, the controller 150 may control the robotic device 160 to align tips of the interventional devices 101 within a predetermined distance range (e.g., 5 pixels) of alignment. Alignment may be performed when a distance between the respective tips of the inner device and the outer device is outside of the predetermined distance range, such as by more than 5 pixels.

In some embodiments, an estimate of physical distances may be used as the metric. For example, detector pixels may be converted to millimeters using standard projection geometry principles and information or a definition of a C-arm geometry. An estimate may be generated based on assuming that the interventional devices 101 are in a particular elevation (between the X-ray detector and the X-ray source) in the projection space (e.g., an iso center). Aligning is explained with respect to S254 in FIG. 2B and with respect to B of FIG. 3.

As another example, the controller 150 may control the robotic device 160 to rotate the interventional devices 101 once the tips are aligned, such as when both of the interventional devices 101 are located in an intersection between at least two branches but are pointing to the wrong branch. Rotating is explained further with respect to S256 in FIG. 2B and with respect to C of FIG. 3.

As yet another example, the controller 150 may also control the robotic device 160 to retract the two interventional devices 101 once the tips are aligned. The interventional devices 101 may be retracted to a target point such as to an intersection between three branches. The fourth interface 159 may be a thumbwheel user interface used to allow the user to indicate the target point with a marking and direct the robotic device 160. Retracting is explained further with respect to S258 in FIG. 2B and with respect to D of FIG. 3. In some embodiments, the system 100 may suggest multiple branching locations as targets based on vasculature tree traversal from a distal target location in the vasculature. The nearest branching location ahead of the interventional devices 101 may be selected as the default.

As a further example, the inner device or the outer device among the interventional devices 101 may be controlled by the robotic device 160 to rotate alone, separately and independently of the other of the outer device or the inner device. The inner device or the outer device may be rotated to align the curvature of the ends of the interventional devices 101 based on the tangent of the tips. Separately rotating is explained further with respect to S260 in FIG. 2B and with respect to E of FIG. 3, and may be useful in the case when a curved catheter appears straight in the x-ray image due to the curve being perpendicular to x-ray plane (i.e., since the X-ray plane is parallel to the X-ray detector).

In some embodiments, a user alert may be issued to the user via a visual guidance system, and the user may be prompted to rotate one of the inner device or the outer device. Alternatively, the user may be provided guidance as to the direction in which the robotic device 160 is moving the interventional devices 101, and where the interventional devices 101 will be stopped by the robotic device 160 based on the controller 150 automatically interpreting images and the state of the robotic device 160.

As another example, the inner device (the guidewire) of the interventional devices 101 may be advanced by the robotic device 160 to a target point. Advancing the inner device (the guidewire) to a target point is explained with respect to S262 in FIG. 2B and with respect to F of FIG. 3.

The controller 150 may also advance the inner device by a distance past the outer device, while keeping the outer device static. The controller 150 may servo-control the robotic device 160 to retract the outer device by a distance relative to the inner device, keeping the inner device static. The controller can also retract the inner device relative to the outer device. The controller 150 may actively advance and retract the inner device relative to the initial position of the tip of the inner device relative to the image. In this way, the controller 150 may anchor the inner device to the vessel/branch. Advancing the inner device past the outer device may be performed when the controller 150 is retracting or rotating the outer device.

The controller 150 may also provide suggestions on the display 180 for the user to show expected motions in a graphical sequence. The motions may be suggested when a list of one or more common motions/maneuvers are presented to a physician based on, for example, geometry of a specified vessel, definition of a target, the specific type(s) of the interventional devices 101, and so on. The motions may be suggested using a trained prediction model using past sequences of motions and the shape of the anatomy of the current subject, as well as the location of the target point in the anatomy of the current subject.

The processor 152 may also provide servo-commands to the robotic device 160 based on the selection of one or more tools either automatically by the controller 150 or based on input from the user via the fourth interface 159. The servo-commands are communicated to the robotic device 160 via the first interface 156.

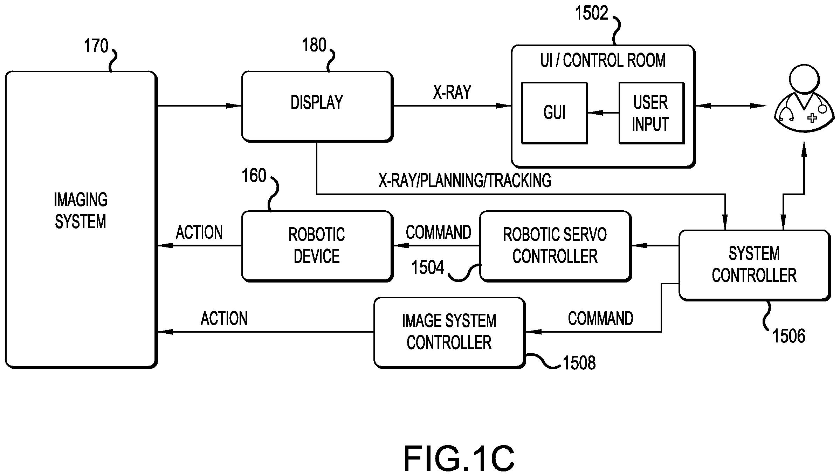

FIG. 1C illustrates another system for control of robotic endovascular devices with fluoroscopic feedback, in accordance with a representative embodiment.

In FIG. 1C, a system includes the system includes the robotic device 160, the imaging system 170 and the display 180. However, instead of the controller 150, separate control systems are provided by the user interface/control room 1502, the robot servo controller 1504, the system controller 1506, and the image system controller 1508. Any of the user interface/control room 1502, the robot servo controller 1504, the system controller 1506 and the image system controller 1508 may include a memory that stores instructions and a processor that executes the instructions to implement the functionality attributed herein to these elements.

The user interface/control room 1502 is used by a user to input instructions to the overall system. The instructions may be input via a graphical user interface such as the display 180, or may be confirmed via the display 180 as the user inputs the instructions via another user input such as a mouse and/or keyboard.

The robot servo controller 1504 receives commands from the system controller 1506, and controls the robotic device 160 based on the commands from the system controller 1506.

The system controller 1506 is the closest functional element to the controller 150 in FIG. 1A and FIG. 1B, and controls the robotic device 160 and the imaging system 170 based on input from the user and fluoroscopic feedback from the imaging system 170. The fluoroscopic feedback may be used to automatically control at least some functions of the robotic device 160 in accordance with various teachings herein.

The image system controller is used to control the imaging system 170 based on instructions from the system controller 1506.

FIG. 2A illustrates a method for control of robotic endovascular devices with fluoroscopic feedback, in accordance with a representative embodiment.

The method of FIG. 2A may be performed by the controller 150, by the system 100 including the controller 150. At S210 of FIG. 2A, image data is received. For example, image data may be received by the controller 150 from the imaging system 170. The receiving at S210 may include receiving image data of an image from the imaging system 170. The image may include a portion of the interventional devices 101 and a branched intersection of a plurality of branches of the anatomical structure. The plurality of branches may include a main branch and a target branch which is branched from the main branch.

At S220, the image data is analyzed. The image data may be analyzed by the controller 150, and specifically by the processor 152 executing instructions from the memory 151 to analyze the image data received at S210. The analyzing at S220 may include segmenting the interventional devices 101 in the image, and/or analyzing the image data to measure geometric characteristics of the outer device and the inner device in the image, based on the branched intersection including the main branch and the target branch in the image. The geometric characteristics may include dimensions and distances of the interventional devices 101 such as an exposed length of the interventional devices 101 visible in the image, width and/or heights of the interventional devices 101 visible in the image, and dimensions and distances of the plurality of branches. The geometric characteristics may include a location of a distal portion of the outer device and a distal portion of the inner device in the image. The geometric characteristics may also include orientations of the distal portion of the outer device and of the distal portion of the inner device in the image. The dimensions, distances and orientations may be parameterized in the analysis at S220, such as based on a count of pixels corresponding to the distances or by angles defining the difference between orientations and predetermined axes. Accordingly, the analyzing at S220 may include measuring at least one of a location or an orientation of a distal portion of the outer device of the interventional devices 101 and of a distal portion of the inner device of the interventional devices 101 in the image, with respect to vasculature including the main branch and the target branch and an intersection of the main branch and the target branch in the image.

At S230, the process of FIG. 2A includes determining a path. The path may be defined by a sequence of predefined motions which correspond to modules of predefined motions stored in a memory (e.g., the memory 151). The path may be determined by the controller 150, and the path may be constructed with reference to the library of predefined motions stored in the memory 151. For example a path may include a suggested set of branches from the anatomy of the current subject.

At S240, the path is displayed on the image. In some embodiments the entire path may be displayed on the image, and in other embodiments less than the entire path may be displayed on the image so that at least a portion of the path is displayed on a display. The path may be superimposed on the branches of the anatomy of the current subject in the images from the imaging system 170 displayed on the display 180. For example, the path may be color-coded to delineate different motions to be made.

At S250, the interventional devices 101 are controlled. The interventional devices 101 may be controlled by the robotic device 160 under instructions from the controller 150, and the control may be one or more of the actions described with respect to FIG. 2B.

The control at S250 may include direct control by the controller 150, or indirect control such as by defining and sending a command to control the robotic device 160 to operate the interventional devices 101. The control is based on the sequence of motions defining a path or/and by a selection by a user of a displayed predefined motion of the sequence. Individual motions of the sequence may be displayed and made selectable by a user, such as via an interactive touch interface or a mouse or keyboard or voice-command user interface.

The method of FIG. 2A may be performed by the system 100, and primarily by the controller 150 controlling the robotic device 160 using the image data received from the imaging system 170. The robotic device 160 is controlled to operate the interventional devices 101 through the path by a sequence of the predefined motions..

FIG. 2B illustrates a method for control of robotic endovascular devices with fluoroscopic feedback, in accordance with a representative embodiment. Although FIG. 2B shows a linear progression of six processes, it should be clear that control of the robotic device 160 by the controller 150 may involve one or more but fewer than all six processes in a variety of different embodiments.

At S252, the method of FIG. 2B includes advancing. The advancing may include controlling the robotic device 160 to operate the interventional devices 101 through the path to advance past a branched intersection. Advancing one or both of the interventional devices 101 is technically more complex than retraction, since advancing presents the challenge of interacting with tissue such as vascular walls. In some embodiments, the interventional devices 101 may be operated to approach a branched intersection but may be operated to stop before reaching the branched intersection.

At S254, the method of FIG. 2B includes aligning. The aligning may include controlling the robotic device 160 to align a distal portion of the outer device and a distal portion of the inner device. The distal portion of the inner device may be a first distal tip and the distal portion of the outer device may be a second distal tip. The aligning may provide coincident distal portions of the interventional devices 101. Aligning may include advancing the catheter tip to the guidewire tip, or retracting the guidewire tip to the catheter tip.

At S256, the method of FIG. 2B includes rotating. The rotating may include controlling the robotic device 160 to rotate the aligned coincident distal portions of the interventional devices 101 to point in a direction toward the target branch in the current view of the images from the imaging system 170.

At S258, the method of FIG. 2B includes retracting. The retracting may include controlling the robotic device 160 to retract the aligned coincident distal portions of the interventional devices 101 to a target point in an image plane of the image. The target point may have a known relationship relative to the target branch. Retracing as in S258 may be performed so that the interventional devices 101 are in a known position for the next motion/maneuver.

At S260, the method of FIG. 2B includes separately rotating the inner device and/or the outer device. The separate rotating at S260 may include controlling the robotic device 160 to separately angularly rotate the inner device and/or the outer device of the interventional devices 101 to align curvatures of the inner device and outer device. The angular rotation at S260 may be performed so that the interventional devices 101 are in a known position for a next motion/maneuver.

At S262, the method of FIG. 2B includes advancing. The advancing may include controlling the robotic device 160 to advance the inner device of the interventional devices 101 to a target point in the target branch.

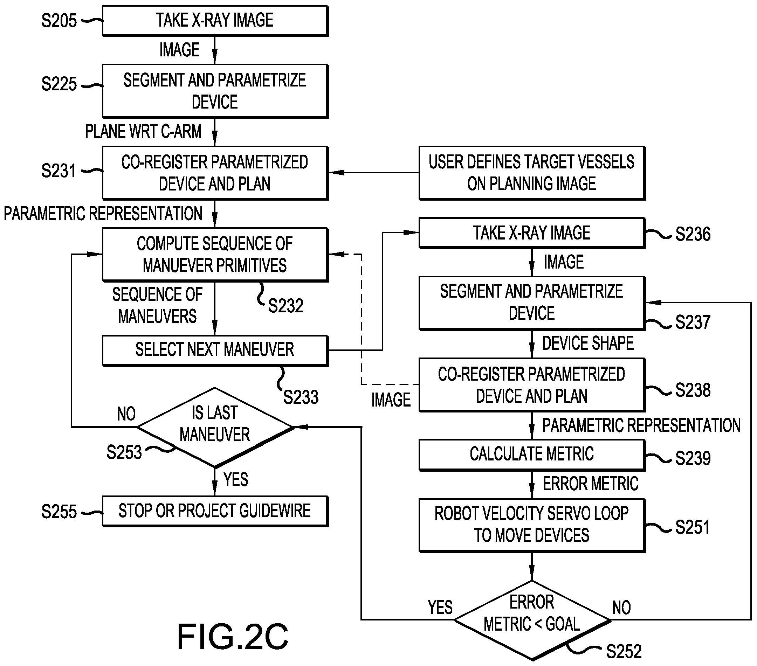

FIG. 2C illustrates another method for control of robotic endovascular devices with fluoroscopic feedback, in accordance with a representative embodiment.

In FIG. 2C, a planning image is taken at S205. The planning image is an X-ray image and may be taken by the imaging system 170. The planning image may be a fluoroscopic image or an angiography image. The planning image may alternatively be a 3D image taken using vessel navigator, for example.

At S225, the interventional devices 101 are located in the planning image, and then segmented and parameterized. The vessel boundaries are segmented from angiography, or the user may define the vessel boundaries directly on the image such as on an interactive display displayed on the display 180.

At S230, the parameterized device is co-registered. The planning image is registered with an image that contains the interventional devices 101. The co-registering may be performed from the same X-ray position. In embodiments using a 3D patient (vessel) model, a 2D to 3D registration may be performed.

At S232, the sequence of motions/maneuvers are computed. The sequence may be computed by the controller 150 in FIG. 1A and FIG. 1B, or by the system controller 1506 in FIG. 1C.

At S233, the next motion/maneuver is selected, and an X-ray image is again taken at S236. At S237, the interventional devices 101 are again segmented and parameterized, and at S238 the parameterization of the interventional devices 101 is co-registered.

At S239, a metric is calculated, and at S251 the robotic device moves the interventional devices 101 via a velocity servo loop.

At S252, a determination is made whether the metric being used is below (or above) the relevant threshold (goal). If the error metric is not below (or above) the relevant threshold (S252 = No), the process returns to S237. If the error metric is below (or above) the relevant threshold (S252 = Yes), a determination is made at S253 as to whether the last motion/maneuver was the final motion/maneuver. If the last motion/maneuver was the final motion/maneuver (S253 = Yes), the process stops or the guidewire is projected at S255. If the last motion/maneuver was not the final motion/maneuver (S253 = No), the process returns to S232.

In the process of FIG. 2C, a user may be enabled to stop the process at any time, such as by pressing a predetermined button or soft key or otherwise entering a command to stop the process. The robotic device controls the interventional devices 101 inside the patient via the velocity servo loop at S251. The interventional devices 101 are visible in the X-rays taken at S205 and S236. Additionally, the metric may be a translation metric such as distance (in pixels) along the current vessel to the center of the target ostium. The parametric representations of the interventional devices 101 created at S225 and S237 may use line segments.

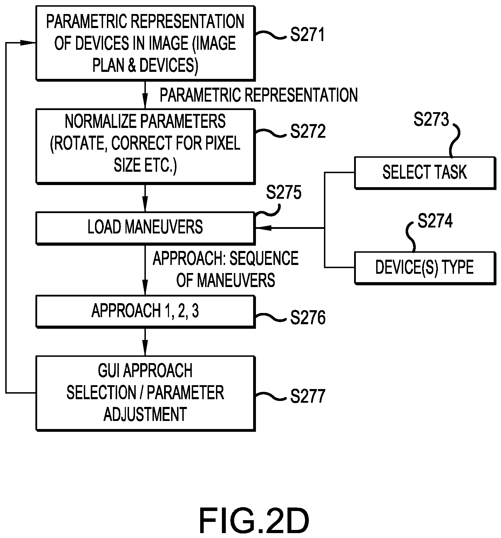

FIG. 2D illustrates another method for control of robotic endovascular devices with fluoroscopic feedback, in accordance with a representative embodiment.

At S271, a parametric representation of the interventional devices 101 in the image is obtained. At S272, the parameters for the interventional devices 101 are normalized. For example, image adjustments used for normalizing the parameters may include rotating the interventional devices 101, correcting for pixel size, and so on.

At S275, the motions/maneuvers are loaded. The motions/maneuvers may be loaded by a selection of task at S273 and identification of the type of the interventional devices 101 at S274.

At S276, a selection of which approach among approaches 1, 2 or 3 is made. At S277, a graphical user interface selection for parameter adjustment is received, and used to identify the parametric representation of the interventional devices 101 at S271.

In FIG. 2D, a user selects the task at S273. A prescribed set of operations for the task may be suggested based on the selection of the task, and the motions/maneuvers for the task may be loaded at S275 based also on the identification of the type of the interventional devices 101 at S274.

As one example, a prescribed set of operations may include A catharize a renal vessel, B cross an aortic aneurysm, C cross the calcific partial occlusion, D catharize the supra aortic vessel, and so on. Some of the operations may be automatically suggested based on the location of the interventional devices 101 and the desired target location in the vasculature. For example, if the interventional devices 101 are in in the iliac artery and the target location is in the renal artery, operations may be automatically suggested for a given type of the interventional devices 101. As an example, measuring the current arterial vessel width and target arterial vessel width may be taken to imply task A. An example of the arterial vessel width might be 100 pixels, and an example of the target arterial vessel might be 60 pixels.

FIG. 3 illustrates motions of an endovascular guidewire and catheter for control of robotic endovascular devices with fluoroscopic feedback, in accordance with a representative embodiment.

The motions in FIG. 3 include advancing in A of FIG. 3, aligning in B of FIG. 3 so that ends of the interventional devices 101 are coincident for the next motion/maneuver, rotating towards a target vessel in the current view in C of FIG. 3, retracting to a location in D of FIG. 3 so that the interventional devices are in a known position for the next motion/maneuver, aligning devices angularly in E of FIG. 3 so that they are in known positions for the next motion/maneuver, and advancing a guidewire to a target in F of FIG. 3. Control methods described herein may use fluoroscopic feedback to robotically and autonomously control a coaxial catheter and guidewire combination for the purpose of assisting in vascular navigation. Individual navigation steps are broken down to discrete maneuvers/motions, which can be executed independently by an operator. For example, an operator may press a joystick button as the fourth interface 159, or may select a vessel as a target branch using an interactive visual interface (e.g., touch screen) on the display 180 of on the fourth interface 159. By providing the predefined motions as a library from which a path of individual motions can be built, controllability may be improved, and paths can be dynamically and optimally developed to account for image interpretation unique to each medical intervention and the complex human anatomy of each subject. As result, an operator is provided an ability to execute high level maneuvers/motions without having to explicitly control each minute motion of the interventional devices 101.

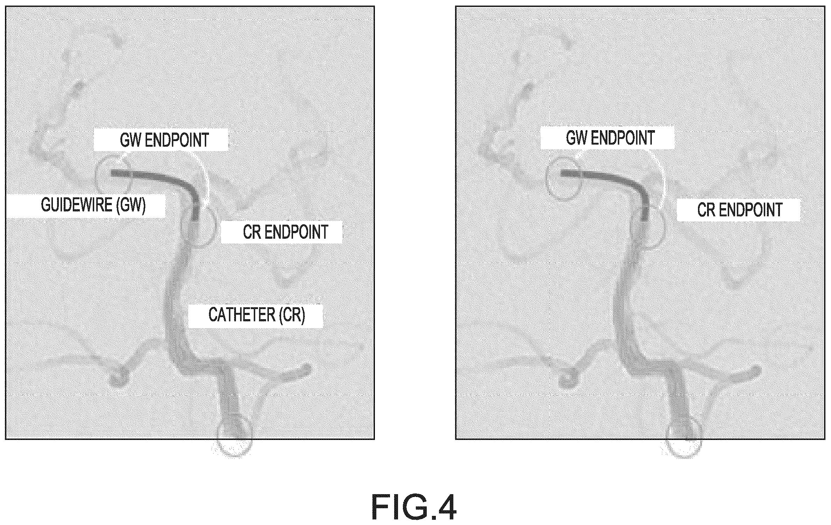

The general control process for each maneuver in A through E of FIG. 3 is generalizable. Error metrics used for control of the robotic device 160 may vary for each motion/maneuver. An example embodiment includes linear alignment of the interventional devices 101, as in B of FIG. 3, which includes adjusting an amount of protrusion of the guidewire so that the tips of both of the interventional devices 101 are coincident on the image. Either the protruding guidewire is retracted towards the static catheter tip or the catheter advances over the static guidewire. Overlapping of tips of the interventional devices 101 are shown on the X-ray images in FIG. 4.

In some embodiments using the teachings of FIG. 3, a display such as the display 180 in FIG. 1A may provide representations of the different predefined motions of at least a part of the sequence for a path through an anatomical structure. The representations may correspond to selectable icons, such as soft keys which are selectable on a touch screen or by a mouse cursor or keyboard. In some embodiments, the representations may be provided in the sequential order. Selections of the representations may result in a command being sent to the robotic device 160.

FIG. 4 illustrates alignment of an endovascular guidewire and catheter for control of robotic endovascular devices with fluoroscopic feedback, in accordance with a representative embodiment.

In FIG. 4, interventional devices 101 are aligned linearly. On the left, a guidewire is retracted to a catheter tip. On the right, the catheter advances over the guidewire towards the guidewire tip. The guidewire tip is labelled as GW endpoint in each view, and the catheter endpoint is labelled CR endpoint in each view.

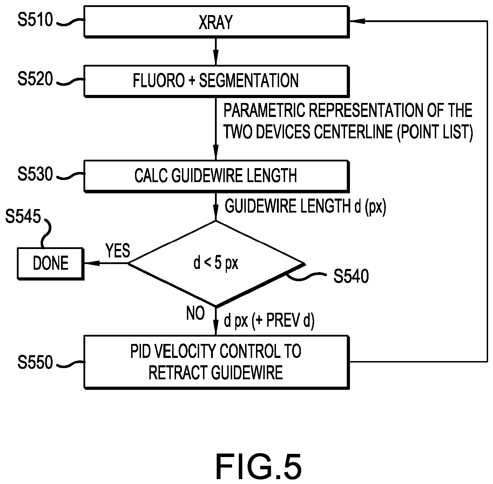

FIG. 5 illustrates a method for control of robotic endovascular devices with fluoroscopic feedback, in accordance with a representative embodiment.

In FIG. 5, a process for driving the guidewire to align with the catheter tip is shown. The controller 150 proceeds through a control loop shown in FIG. 5.

At S510, X-ray images are collected. The interventional devices 101 are segmented in each fluoroscopic image at S520. Parametric representations of each of the interventional devices 101 are provided as a centerline in each image.

At S530, the process of FIG. 5 includes calculating an exposed length of the guidewire, such as by counting pixels showing the guidewire in the image. At S540, the process of FIG. 5 includes comparing the calculated exposed length of the guidewire with a predetermined distance threshold, such as 5 pixels.

If the exposed length of the guidewire is within the threshold (S540 - Yes), the process of FIG. 5 ends at S545. If the exposed length of the guidewire is not within the threshold (S540 = No), the controller 150 controls the robotic device 160 via PID velocity control to retract the guidewire at S550, and the process returns to S510.

In FIG. 5, fluoroscopy images are continuously collected, and device segmentation is performed to parametrize the interventional devices 101 in each image using line segment representations. The segmentation may be performed using artificial intelligence. The exposed length of visible guidewire in pixels (px) is then used as an error metric in a robotic device PID control loop to retract the position of the guidewire relative to the static catheter. In other words, the guidewire is retracted so that the parametrized exposed length of the guidewire decreases if the exposed length of the guidewire in the fluoroscopic image is greater than a predetermined distance threshold. The servo motion continues until a tolerance error is satisfied. As an alternative to the process in FIG. 5, a catheter may be advanced over the static guidewire until the guidewire and the catheter are aligned, in which scenario the guidewire exposed length is minimized by servo-controller the catheter.

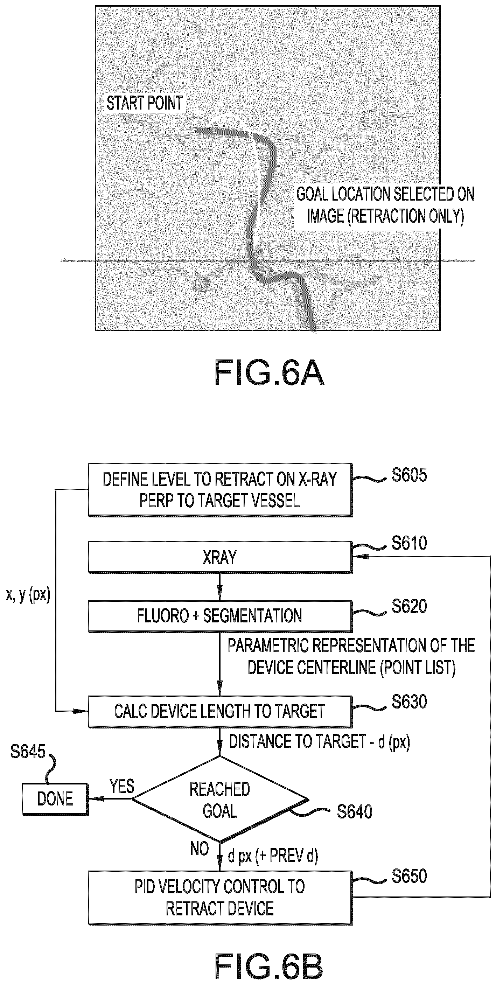

FIG. 6A illustrates a graphical user interface for control of robotic endovascular devices with fluoroscopic feedback, in accordance with a representative embodiment.

In FIG. 6A, a graphical user interface is provided for selecting a location as a target location for retracting the interventional devices 101. A user may be provided an ability to select a location on a touch screen on the display 180, or using a cursor controlled by a mouse as the fourth interface 159.

FIG. 6B illustrates a method for control of robotic endovascular devices with fluoroscopic feedback, in accordance with the graphical user interface in FIG. 6A.

In FIG. 6B, the operator selects the location on an image on the graphical user interface in FIG. 6A. At S605, the process of FIG. 6B starts with the user defining a level to retract on the X-ray (fluoroscopic) image to the target vessel, and the process proceeds to S630 to calculate the exposed length from the interventional devices 101 as a distance to target. At S640, the process of FIG. 6B includes determining whether a goal is reached in the retracting. If the goal is reached (S640 = Yes), the process of FIG. 6B concludes at S645. Otherwise ((S640 = No), the controller 150 provides PID velocity control to the robotic device 160 to retract the interventional devices 101, and the process returns to S610.

At S610, a new X-ray image is obtained. At S620, the interventional devices 101 are segmented in the image obtained at S610. The parametric representation of the interventional devices 101 is again provided as a device centerline and the exposed length from the interventional devices 101 to the target is again calculated at S630 as a distance to target.

In FIG. 6B, the location is a target location for retracting the interventional devices 101, i.e., an indication of where the interventional devices 101 are to be retracted. In case of a joystick input, a double axis input (a thumb joystick) may be used to control the elevation along the normal of the line, and the rotation of the line on the image as shown in FIG. 6A. The horizontal line is intersected with the parametric description of the interventional devices 101 in the image, and the intersection of the horizontal line and the representation of the interventional devices 101 in the image is the target point. The control method in FIG. 6B is used to retract one or multiple of the interventional devices 101 to the target point within some tolerance.

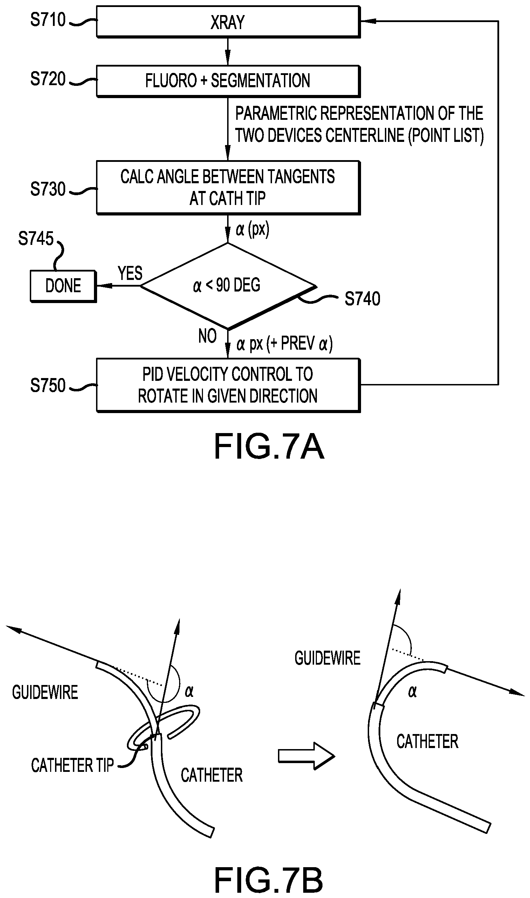

FIG. 7A illustrates a method for control of robotic endovascular devices with fluoroscopic feedback, in accordance with a representative embodiment.

FIG. 7A, a method for aligning two curved devices of the interventional devices 101 in 3D space from 2D fluoroscopy projections is depicted. A perfect 3D alignment of two curved devices in 3D space from 2D fluoroscopy projections is technically challenging and impractical. When the static device (usually the catheter) among the interventional devices 101 is visibly curved in the image, it is difficult to automatically rotate the dynamic device (usually the curved guidewire) such that the curvature of the dynamic device lays on the same side of the curvature of the static device. This is shown in FIG. 7B. The control method in FIG. 7A uses an angle servo metric built from tangents of the distal portions of each of the interventional devices 101, with the reference axis tangent to the catheter distal portion. The magnitude of the angle between the tangent line of the guidewire and the reference axis indicates the direction of rotation after a few small rotations that will bring the guidewire to point in the same direction as the catheter. The algorithm may stop when the angle is less than 180 degrees. Special care may be taken around the 180 degree mark due to a singularity and a discontinuity, such as when rotation is continued until the angle is below 175 degrees. In cases when closest alignment of curvatures is desired, corresponding to the biggest curve, the rotation can continue until the guidewire tangent is the closest possible to 90 degrees. When the angle exceeds 180 degrees, the method may include rotating, or displaying on the display 180 a proposed rotation to be triggered by the user of the inner device toward the reference axis until the angle between the inner tangent of the inner device and the reference axis is less than 180 degrees. Alternatively, when the angle exceeds 180 degrees may include including in the sequence of motions a rotation of the inner device toward the reference axis until the angle between the inner tangent of the inner device and the reference axis is less than 180 degrees.

At S710, an X-ray image is obtained, and the interventional devices 101 in the image are segmented at S720. At S730, an angle between the tangents at the distal portions of each of the interventional devices 101 is calculated. At S740, the process of FIG. 7A includes comparing the calculated angle with a threshold. The threshold is ninety degrees in FIG. 7A. If the calculated angle between an outer tangent of the distal portion of the outer device and an inner tangent of the distal portion of the inner device of the interventional devices 101 is below ninety degrees (S740 = Yes), the process ends at S745. If the calculated angle is not below ninety degrees (S740 = No), PID velocity control is provided to the robotic device 160 to rotate the interventional devices 101 in a given direction, and the process returns to S710.

When a curved catheter appears straight in the X-ray image, such that the curve is perpendicular to the X-ray plane, the operator may be prompted to change the X-ray perspective, or rotate the catheter to expose the curvature. When the catheter is straight, the objective may be a straight alignment or mirroring of the guidewire about the catheter. Additionally, the target reference angle may also be tuned to uniquely shaped catheters. In some cases, a more refined alignment is desired, in which case the C-arm of the imaging system 170 is moved to a new pose to provide another perspective for servo alignment.

FIG. 7B illustrates a graphical user interface for control of robotic endovascular devices with fluoroscopic feedback, in accordance with the method in FIG. 7A.

As shown in FIG. 7B, the distal portions of the interventional devices 101 are pointing in different directions, so one or both of the interventional devices 101 are rotated until an angle between an outer tangent of the distal portion of the outer device and an inner tangent of the distal portion of the inner device is below a predetermined threshold.

In some other embodiments, a guidewire may be actively anchored to a location. For example, when the guidewire is in a side branch while the catheter is in the proximal larger branch, the motion of the catheter may begin to retract the guidewire. This presents a challenge in manual manipulation because even a skilled professional needs to manipulate both of the interventional devices 101 in four degrees of freedom simultaneously with only two hands. Using locations of the interventional devices 101 in images, the robotic device 160 may counteract this by advancing the guidewire relative to the catheter such that the guidewire maintains its depth in the side branch. This is accomplished by active guidewire servoing, with the feedback metric being the location of the tip of the guidewire inside the side vessel. When the tip of the guidewire is retracting due to external forces such as catheter motion, the guidewire may be automatically advanced in proportional to the retraction amount from the initial position. This minimizes the movement of guidewire escaping the cannulated side branch. In other embodiments, the guidewire may be further extended into the side branch to provide additional stabilization.

In some other embodiments, a guidewire position in a cannulated vessel may be slipping, and when this is recognized, the movement of the catheter may be stopped automatically and a new maneuver suggested. Additionally, when the catheter is not rotated in the correct orientation to be facing the opposite direction of the cannulated vessel, the incorrect orientation may be detected and communicated to the user along with a suggested correction. Alternatively, the correction may be automatically implemented once the incorrect orientation is detected. As a result, the guidewire may be actively anchored to the vessel by automatically correcting the catheter orientation.

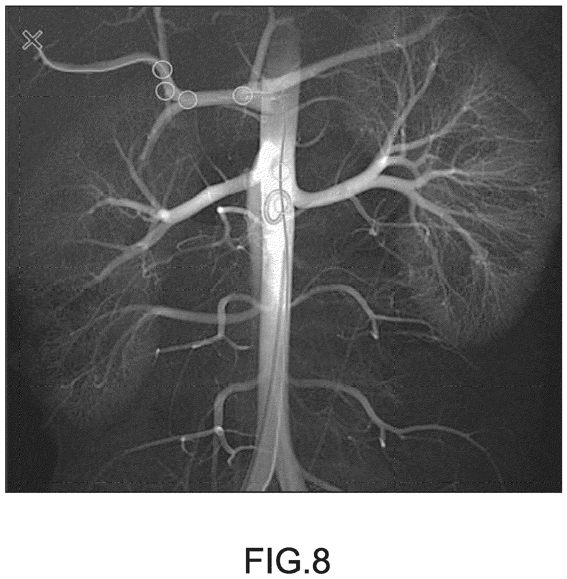

FIG. 8 illustrates a user interface for control of robotic endovascular devices with fluoroscopic feedback, in accordance with a representative embodiment.

In FIG. 8, a user interface defines a navigation target on a vasculature path. The user interface presents a single axis input for selecting a target anatomical structure. To simplify the target point selection by the user, the operator is presented with a scrolling single degree of freedom input option that steps through the preplanned vascular path that navigates branches of the vasculature starting at the largest vessel - typically most proximal to vascular access site towards the target. The scrolling single degree of freedom may be provided via, for example, a scroll wheel, slider, or knob. When the target navigation location is known, a trivial reverse tree traversal may be applied to create a shortest path from the large vessel or current location to the target, which in many cases will be the only path from the large vessel or current location to the target. This continuous path is then traversed by the user input cursors as a 1-dimmensional space in discrete steps, either preset distance units or bifurcation locations. For example, scrolling to 50% of the path length may be used to control the robotic device 160 to move the interventional devices 101 one-half of the path from the starting point. The selection may be implemented using a 3D model, a 2D image, or a derived image. Alternatively, the starting point may be the closest branch to the end of the most distant device. The most distance device is usually, but not always, the guidewire.

Input from a user may also be provided in the form of planning. For example, ring landmarks may be provided in a vessel navigation tool to define which ostea to cannulate. Planning may also be provided in the form of data from prior procedures, such as in interventional oncology where procedures may repeatedly involve the same location in the body.

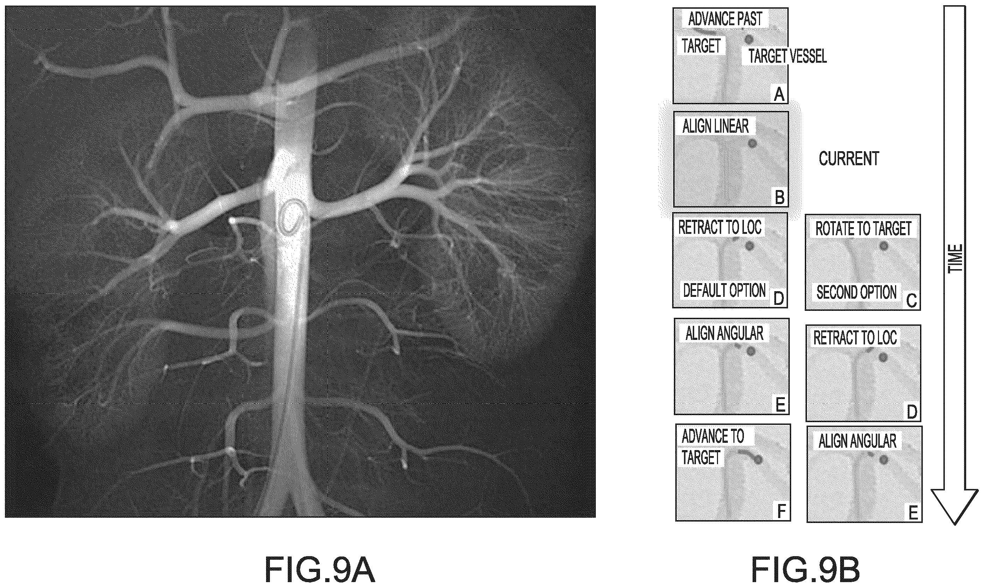

FIG. 9A illustrates a user interface for control of robotic endovascular devices with fluoroscopic feedback, in accordance with a representative embodiment. FIG. 9B illustrates a sequence of motions with an option to change or accept, for control of robotic endovascular devices with fluoroscopic feedback, in accordance with the user interface in FIG. 9A.

FIG. 9A presents a user interface for communicating a current maneuver and sequence of future maneuvers with an option to change or accept. The system 100 can present an expected maneuvers in a graphical sequence to the physician on the display 180, with highlights for each expected motion/maneuver including the motion/maneuver, which is currently active, and including the expected sequence of next motions/maneuvers. The physician is provided an option to select the motions/maneuvers that are recommended to be executed next. The maneuver list may be limited to the currently viable maneuvers, see. For example if the guidewire is already aligned with the catheter, the alignment maneuver is not presented in a selection interface. In addition a group of maneuvers can be presented. The transparent interface in FIG. 9B is extremely important for communicating robotic device 's intention and resulting behavior to the physician.

FIG. 9B also presents a user interface for communicating a current maneuver and sequence of future maneuvers with an option to change or accept. In FIG. 9B, the next N number of maneuvers may be suggested based on previous sequence of observed maneuvers executed by the robotic device 160. The user interface in FIG. 9B may use a sequence prediction technique to generate complex sequences where a future sequence is generated as an output based on same general characteristics as other sequences in the data. The input is a sequence of the maneuvers represented by indexes (A,B,...) and the output is the most likely maneuver to follow (D). This process may be repeated by looping the suggested output and the previous sequence into the prediction network to generate a sequence of likely future maneuvers (D,E,F). The user may accept the next suggested maneuver or scrolls through candidate maneuvers which are sorted based their future execution probability.

In some embodiments using the teachings of FIG. 9B, a display such as the display 180 in FIG. 1A may provide representations of the different predefined motions of at least a part of the sequence for a path through an anatomical structure. The representations may correspond to selectable icons, such as soft keys which are selectable on a touch screen or by a mouse cursor or keyboard. In some embodiments, the representations may be provided in the sequential order. Selections of the representations may result in a command being sent to the robotic device 160. In FIG. 9B, the predefined motions for an entire path may be shown in the column on the left, and the predefined motions remaining for the interventional devices 101 may be shown in the column on the right. Additionally, as shown, the predefined motion which is currently being performed may be designated by words, highlighting, or otherwise so that a user can quickly identify which predefined motion in a sequence is being performed currently.

The controller 150 may provide a servo-command based on the execution or selection of one of the tools shown in FIG. 9B. The selection of the tools in FIG. 9B may be by the controller 150 or by the user via the fourth interface 159, and the servo-command may be communicated to the robotic device 160 via the first interface 156.

In embodiments with X-ray biplane imaging, the target reference point can be defined in 3D using triangulation. In this case the tip of the interventional devices 101 is continuously triangulated using epipolar geometry techniques for stereo reconstruction borrowed from computer vision. In cases where the two X-ray imaging sources are not well calibrated, independent target references are used, and each one is met for the goal to be reached. These embodiments may be useful for rotating the interventional devices 101 to align their respective curvatures.

In some embodiments, open-loop control may be incorporated. To minimize X-ray use, open-loop control using pre-planned motions can be interweaved with the image feedback maneuvers. Open-loop control may be used initially as a first pass, even when precision requires endpoint sensing (e.g., X-ray) feedback. For example, to align ends of the guidewire and catheter, a pre-calibrated wire/catheter model may be used to retract the guidewire so the ends are coincident. However, when the estimated difference is within some tolerance such as 2cm, the X-ray imaging resumes and is used for servoing to complete the maneuver.

In some embodiments, collimation around a guidewire may be synchronized. Since maneuvers are performed semi-automatically, the area around the interventional devices 101 is the region required for feedback. Synchronization of collimation during servo motions may be performed based on segmentation of the interventional devices 101 that is to be controlled. A bounding box of the distal portion to be controlled may be created. Adjustments of the collimation are performed based on expected motion of the interventional devices 101 per the current maneuver sequence.

In some embodiments, a guidewire may be loaded to the tip of catheter. For exchanging interventional devices 101 and for roughly calibrating lengths of the interventional devices 101, the guidewire (or any other device) may be advanced after installation in the catheter until the tip becomes visible in the X-ray view. The guidewire may then be retracted until both tips are aligned. The result may serve as a "homing" calibration which can be revisited and which can enable some open-loop coordinate motions between the interventional devices 101. The calibration may be useful in cases when the guidewire is difficult to visualize through an opaque catheter.

In some embodiments, artificial intelligence (AI) methods may be used for reinforcement learning. AI methods may be more tractable if they operate on a lower dimensional space, and this is particularly relevant in Reinforcement Learning techniques where the actions can be limited to a few operations such as the discrete maneuvers described herein (i.e., for output actions for a reinforcement learning agent to reach a goal). Although direct 4-degree-of-freedom control of the robotic device 160 as actions are theoretically possible, a reinforcement learning network may require learning a significant number of micro-steps in order to achieve a particular outcome, which in practice requires a large number of trials. As a result, composing actions of the reinforcement learning agent from relatively higher-level actions is preferable, and are useful in the context of the teachings herein.

In some embodiments, the interventional devices 101 may be modified such as by adding unique properties of the interventional devices 101 to augment the target points for the servo methods. The interventional devices which may be appropriate for modification include stents and balloons, and the unique properties which may be added or modified include elasticity, geometry model (X-ray visible or not), the estimated interaction between the interventional devices 101 such as expected curvature, etc. For example, when the interventional devices 101 have a marker which is mostly opaque, the actual geometry may be registered with the visible marker and superimposed on the invisible sections of the image. The augmentation may be used as input into the maneuver control methods described herein.

In some embodiments, discrete markers may be provided for the interventional devices 101 for velocity control. When a guidewire has markers it is possible to estimate velocity of the markers along the guidewire to improve out of plane ambiguity when approaching distance alignment. This may be performed by retracting at constant velocity when the markers on the guidewire appear in the X-ray image but are not moving.

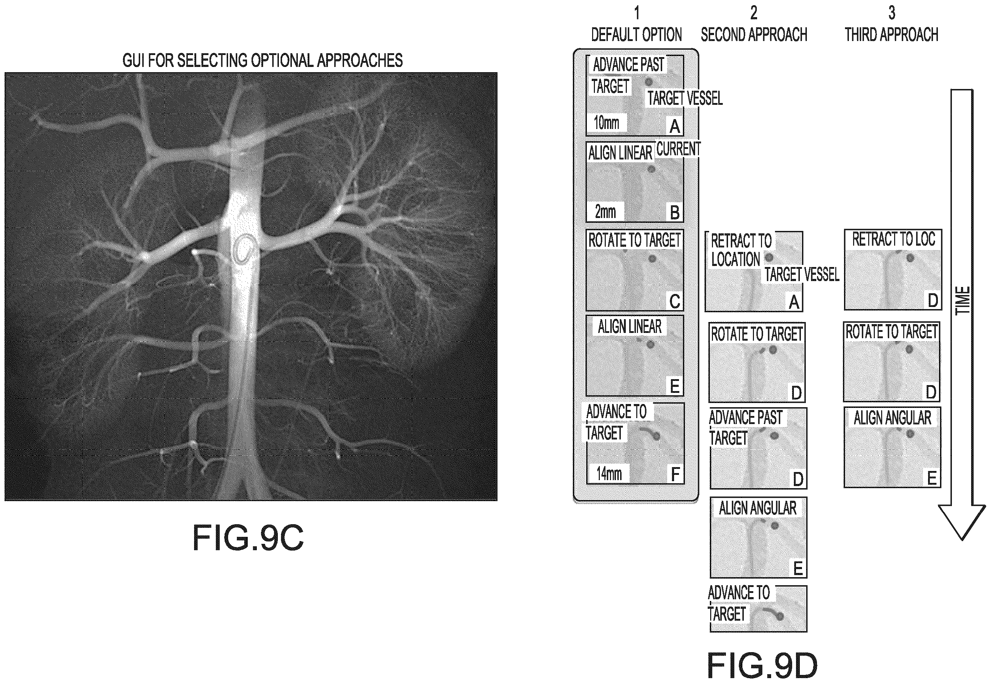

FIG. 9C illustrates another user interface for control of robotic endovascular devices with fluoroscopic feedback, in accordance with a representative embodiment.

FIG. 9C presents a user interface for communicating a current maneuver and sequence of future maneuvers with an option to change or accept. The system 100 can present sets of potential motions/maneuvers in graphical sequences to the physician on the display 180, with highlights for each expected motion/maneuver, including the motion/maneuver which is currently active and including the expected sequence of next motions/maneuvers for the set. The physician may be provided an option to select the set, and then the motions/maneuvers in the set that are recommended to be executed next. The maneuver list may be limited to the currently viable maneuvers. For example if the guidewire is already aligned with the catheter, the alignment maneuver is not presented in a selection interface. In addition a group of maneuvers can be presented. The transparent interface in FIG. 9D may be important for communicating the expected acts of the robotic device 160 and the resulting behavior of the interventional devices 101 to the physician.

FIG. 9D illustrates a sequence of motions with an option to change or accept, for control of robotic endovascular devices with fluoroscopic feedback, in accordance with the user interface in FIG. 9C.

FIG. 9D also presents a user interface for communicating a current maneuver and sets of motions/maneuvers and sequences of future maneuvers with option to change or accept. In FIG. 9D, the next N number of maneuvers in each set may be suggested based on the previous sequence of observed maneuvers executed by the robotic device 160. In FIG. 9D, three separate approaches are shown as a First Approach, a Second Approach and a Third Approach.

The user interface in FIG. 9D may use a sequence prediction technique to generate complex sequences wherein a future sequence is generated as an output based on same general characteristics as other sequences in the data. The input is a sequence of the maneuvers represented by indexes (A,B,...) and the output is the most likely maneuver to follow (D). This process may be repeated by looping the suggested output and the previous sequence into the prediction network to generate a sequence of likely future maneuvers (D,E,F). The user may accept the next suggested maneuver or scrolls through candidate maneuvers which are sorted based their future execution probability.

In some embodiments using the teachings of FIG. 9C and FIG. 9D, a display such as the display 180 in FIG. 1A may provide representations of three different options for predefined motions comprising three different approaches. Each of the different options may correspond to different sets and/or sequences of predefined motions to use for at least a part of the sequence for a path through an anatomical structure. Each set of predefined motions corresponding to the options, and each of the predefined motions in each set, may correspond to different selectable representations such as different soft keys for different icons. The icons may be selectable on a touch screen or by a mouse cursor or keyboard. In some embodiments, the representations may be provided in the same sequential order as in the options. Selections of the representations may result in a command being sent to the robotic device 160. In FIG. 9D, the predefined motions for a default path may be shown in the column on the left, and the predefined motions for two alternative approaches may be shown in the middle column and the right-most column.. Additionally, the predefined motion which is currently being performed may be designated by words, highlighting, or otherwise so that a user can quickly identify which predefined motion in a sequence is being performed currently.

FIG. 10 illustrates a computer system, on which a method for control of robotic endovascular devices with fluoroscopic feedback is implemented, in accordance with another representative embodiment.

Referring to FIG.10, the computer system 1000 includes a set of software instructions that can be executed to cause the computer system 1000 to perform any of the methods or computer-based functions disclosed herein. The computer system 1000 may operate as a standalone device or may be connected, for example, using a network 1001, to other computer systems or peripheral devices. In embodiments, a computer system 1000 performs logical processing based on digital signals received via an analog-to-digital converter.

In a networked deployment, the computer system 1000 operates in the capacity of a server or as a client user computer in a server-client user network environment, or as a peer computer system in a peer-to-peer (or distributed) network environment. The computer system 1000 can also be implemented as or incorporated into various devices, such as a workstation that includes the controller 150 in FIG. 1A, a stationary computer, a mobile computer, a personal computer (PC), a laptop computer, a tablet computer, or any other machine capable of executing a set of software instructions (sequential or otherwise) that specify actions to be taken by that machine. The computer system 1000 can be incorporated as or in a device that in turn is in an integrated system that includes additional devices. In an embodiment, the computer system 1000 can be implemented using electronic devices that provide voice, video or data communication. Further, while the computer system 1000 is illustrated in the singular, the term "system" shall also be taken to include any collection of systems or sub-systems that individually or jointly execute a set, or multiple sets, of software instructions to perform one or more computer functions.

As illustrated in FIG. 10, the computer system 1000 includes a processor 1010. The processor 1010 may be considered a representative example of the processor 152 of the controller 150 in FIG. 1B and executes instructions to implement some or all aspects of methods and processes described herein. The processor 1010 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 1010 is an article of manufacture and/or a machine component. The processor 1010 is configured to execute software instructions to perform functions as described in the various embodiments herein. The processor 1010 may be a general-purpose processor or may be part of an application specific integrated circuit (ASIC). The processor 1010 may also be a microprocessor, a microcomputer, a processor chip, a controller, a microcontroller, a digital signal processor (DSP), a state machine, or a programmable logic device. The processor 1010 may also be a logical circuit, including a programmable gate array (PGA), such as a field programmable gate array (FPGA), or another type of circuit that includes discrete gate and/or transistor logic. The processor 1010 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 1000 further includes a main memory 1020 and a static memory 1030, where memories in the computer system 1000 communicate with each other and the processor 1010 via a bus 1008. Either or both of the main memory 1020 and the static memory 1030 may be considered representative examples of the memory 151 of the controller 150 in FIG. 1B, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 1020 and the static memory 1030 are articles of manufacture and/or machine components. The main memory 1020 and the static memory 1030 are computer-readable mediums from which data and executable software instructions can be read by a computer (e.g., the processor 1010). Each of the main memory 1020 and the static memory 1030 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 1000 further includes a video display unit 1050, such as a liquid crystal display (LCD), an organic light emitting diode (OLED), a flat panel display, a solid-state display, or a cathode ray tube (CRT), for example. Additionally, the computer system 1000 includes an input device 1060, such as a keyboard/virtual keyboard or touch-sensitive input screen or speech input with speech recognition, and a cursor control device 1070, such as a mouse or touch-sensitive input screen or pad. The computer system 1000 also optionally includes a disk drive unit 1080, a signal generation device 1090, such as a speaker or remote control, and/or a network interface device 1040.

In an embodiment, as depicted in FIG. 10, the disk drive unit 1080 includes a computer-readable medium 1082 in which one or more sets of software instructions 1084 (software) are embedded. The sets of software instructions 1084 are read from the computer-readable medium 1082 to be executed by the processor 1010. Further, the software instructions 1084, when executed by the processor 1010, perform one or more steps of the methods and processes as described herein. In an embodiment, the software instructions 1084 reside all or in part within the main memory 1020, the static memory 1030 and/or the processor 1010 during execution by the computer system 1000. Further, the computer-readable medium 1082 may include software instructions 1084 or receive and execute software instructions 1084 responsive to a propagated signal, so that a device connected to a network 1001 communicates voice, video or data over the network 1001. The software instructions 1084 may be transmitted or received over the network 1001 via the network interface device 1040.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, control of robotic endovascular devices with fluoroscopic feedback enables automatic and consistent wire manipulation assistance. The wire manipulation assistance may reduce risks in medical interventions. An examples of the risks reduced by the subject matter described herein include the risk of perforating delicate vascular walls, which may result in fatal complications. Another example of the risks reduced by the subject matter described herein include the risks presented by stroke treatment (thrombectomy), where timely treatment by skilled professional is essential but not always available.

As set forth above, endovascular intervention workflow may be improved by enabling repetitive maneuvers of devices inside vessels to be executed by robotic means with high precision and speed. A library of motions that can be sequenced together to execute a higher level autonomous task may be provided for dynamic path determination when needed, such as for automatic navigation to deposit an intravascular device into a vascular branch.

Although control of robotic endovascular devices with fluoroscopic feedback has been described with reference to several exemplary embodiments, it is understood that the words that have been used are words of description and illustration, rather than words of limitation. Changes may be made within the purview of the appended claims, as presently stated and as amended, without departing from the scope and spirit of control of robotic endovascular devices with fluoroscopic feedback in its aspects. Although control of robotic endovascular devices with fluoroscopic feedback has been described with reference to particular means, materials and embodiments, control of robotic endovascular devices with fluoroscopic feedback is not intended to be limited to the particulars disclosed; rather control of robotic endovascular devices with fluoroscopic feedback extends to all functionally equivalent structures, methods, and uses such as are within the scope of the appended claims.

## Claims

1. A controller (150) for controlling a robotic device (160) configured to operate an interventional device (101) into an anatomical structure of a subject, the interventional device (101) comprising an outer device and an inner device movably positioned within the outer device, the
controller (150) comprising:
a memory (151) that stores instructions and a library of a plurality of modules of predefined motions for navigating through anatomical structures; and
a processor (1010) that executes the instructions, wherein, when executed by the processor (1010), the instructions cause the controller (150) to:
receive, from an imaging device, image data from an image of a portion of the interventional device (101) and a branched intersection of a plurality of branches of the anatomical structure, including a main branch and a target branch which is branched from the main branch;
analyze the image data to measure at least one of a location or an orientation of a distal portion of the outer device and of a distal portion of the inner device in the image, with respect to vasculature including the main branch and the target branch and an intersection of the main branch and the target branch in the image;
determine, based on analyzing the image data, a path for the interventional device (101) through the anatomical structure to a target using the modules of predefined motions stored in the memory (151), the path being defined by a sequence of predefined motions; and
display (180) at least a portion of the path on a display (180) such that the predefined motions of this portion are represented.

2. The controller (150) of claim 1, wherein the instructions further cause the controller (150) to:
control, based on the sequence or on a selection by a user of a displayed predefined motion of the sequence, the robotic device (160) to operate the interventional device (101) through at least a portion of the path determined by the sequence or selection.

3. The controller (150) of claim 1 or 2, wherein the instructions further cause the controller (150) to:
display (180) at least a portion of the path on a display (180) such that the predefined motions of this portion are represented according to their order in the sequence.

4. The controller of any of previous claims, wherein the display (180) comprises future expected or recommended predefined motions within the sequence.

5. The controller of claim 4, wherein the display (180) further comprises current and past predefined motions within the sequence.

6. The controller of claim 5, wherein the instructions further cause the controller (150) to highlight the predefined motion which is currently active.

7. The controller (150) of any of claim 1 to 6, wherein the instructions further cause the controller (150) to:
determine, based on analyzing the image data, a plurality of future recommended paths for the interventional device (101) through the anatomical structure using the modules of predefined motions stored in the memory (151), each future recommended path being defined by a sequence of predefined motions; and
display (180) representations of at least portions of the plurality of future recommended paths, each representation of a path comprising predefined motions of at least a part of the corresponding sequence.

8. The controller of claim 7, wherein the instructions further cause the controller (150) to provide an option to the user to change and/or accept a recommended path or predefined motions.

9. The controller (150) of any of previous claims, wherein the predefined motions include at least one of:
aligning the distal portion of the outer device with the distal portion of the inner device to provide coincident distal portions of the interventional device (101);
rotating aligned coincident distal portions of the interventional device (101) to point in a direction toward the target branch; and
retracting the aligned coincident distal portions of the interventional device (101) to a target point in an image plane of the image, wherein the target point has a known relationship relative to the target branch.

10. The controller (150) of claim 9, wherein aligning the distal portion of the outer device with the distal portion of the inner device comprising retracting the inner device into the outer device, wherein the instructions further cause the controller (150) to implement said retracting of the inner device into the outer device of the corresponding predefined motion, comprising:
determine an exposed length in pixels of the inner device extending from the distal portion of the outer device;
determine an error metric based on the exposed length of the inner device, and
retract the inner device relative to the outer device using the error metric in a proportional-integral-derivative (PID) control loop, thereby decreasing the exposed length of the inner device until a tolerance error is satisfied.

11. The controller (150) of claim 9, wherein the instructions further cause the controller (150) to implement said retracting of the aligned coincident distal portions of the interventional device (101) to the target point of the corresponding predefined motion, comprising:
receive selection of a location of the target point in the image of the portion of the interventional device (101) and the branched intersection;
measure a length in pixels of the interventional device (101) extending beyond the location of the target point;
initially retract the interventional device (101) by shortening the length in pixels at a first velocity when the length in pixels exceeds a predetermined distance; and
further retract the interventional device (101) by shortening the length in pixels at a second velocity when the length in pixels no longer exceeds the predetermined distance, wherein the second velocity is slower than the first velocity.

12. The controller (150) of any of previous claims, wherein the instructions further cause the controller (150) to:
separately rotate the inner device and the outer device to align curvatures of the inner device and the outer device.

13. The controller (150) of claim 12, wherein the instructions further cause the controller (150) to:
identify an outer tangent to a curve of the outer device as a reference axis;
identify an inner tangent to a curve of the inner device;
determine an angle between an inner tangent of the inner device and the reference axis;
separately rotate the inner device and the outer device to align curvatures of the inner device and the outer and, when the angle exceeds 180 degrees rotate the inner device toward the reference axis until the angle between the inner tangent of the inner device and the reference axis is less than 180 degrees.

14. The controller (150) of claim 12 or 13, wherein the instructions causing the controller (150) to implement said separate rotation of the inner device and the outer device to align curvatures of the inner device and the outer device, further cause the controller (150) to:
when the curve of the outer device is initially not visible in the image, change a perspective of the imaging device or rotate the outer device until the curve is exposed in the image.

15. A system (100) for controlling a robotic device (160) configured to operate an interventional device (101) into an anatomical structure of a subject to a target, the interventional device (101) comprising an outer device and an inner device movably positioned within the outer device, the system (100) comprising:
an imaging interface configured to receive image data corresponding to an image from an imaging device, the image including a portion of the interventional device (101) and a branched intersection of a plurality of branches of the anatomical structure, including a main branch and a target branch which is branched from the main branch;
a user interface configured to receive input from a user;
an interface arranged to connect the system (100) with a robotic device (160) for operating the interventional device (101) into the anatomical structure of the subject;
a robotic device (160) controller (150) of any of claims 1 to 14, the robotic device (160) controller (150) being further configured to control, based on the sequence or on a selection by a user of a displayed predefined motion, the robotic device (160) to operate the interventional device (101) through at least a portion of the path determined by the sequence or selection; and
a display (180) configured to display (180) at least the branch intersection of the plurality of branches of the anatomical structure, the distal portion of the outer device and the distal portion of the inner device of the interventional device (101), while the robotic device (160) controller (150) controls the robotic device (160).

## Patentansprüche

1. Steuereinheit (150) zum Steuern einer Robotervorrichtung (160), die dazu konfiguriert ist,
eine interventionelle Vorrichtung (101) in eine anatomische Struktur eines Subjekts einzuführen, wobei die interventionelle Vorrichtung (101) eine äußere Vorrichtung und eine innere Vorrichtung umfasst, die beweglich innerhalb der äußeren Vorrichtung positioniert ist, wobei die Steuereinheit (150) Folgendes umfasst:
einen Speicher (151), der Anweisungen und eine Bibliothek einer Vielzahl von Modulen vordefinierter Bewegungen zum Navigieren durch anatomische Strukturen speichert; und
einen Prozessor (1010), der die Anweisungen ausführt, wobei die Anweisungen, wenn sie vom Prozessor (1010) ausgeführt werden, die Steuereinheit (150) zu Folgendem veranlassen:
Empfangen von Bilddaten von einem Bild eines Abschnitts der interventionellen Vorrichtung (101) und einer verzweigten Schnittstelle einer Vielzahl von Ästen der anatomischen Struktur, einschließlich eines Hauptasts und eines Zielasts, der vom Hauptast abzweigt, von einer Bildgebungsvorrichtung;
Analysieren der Bilddaten, um mindestens eine Stelle oder Ausrichtung eines distalen Abschnitts der äußeren Vorrichtung und eines distalen Abschnitts der inneren Vorrichtung im Bild in Bezug auf ein Gefäßsystem einschließlich des Hauptasts und des Zielasts und eines Schnittpunkts des Hauptasts und des Zielasts im Bild zu messen;
Bestimmen, basierend auf dem Analysieren der Bilddaten, eines Pfads für die interventionelle Vorrichtung (101) durch die anatomische Struktur zu einem Ziel unter Verwendung der im Speicher (151) gespeicherten Module vordefinierter Bewegungen, wobei der Pfad durch eine Abfolge vordefinierter Bewegungen definiert ist; und
Anzeigen (180) mindestens eines Abschnitts des Pfads auf einer Anzeigeeinrichtung (180), sodass die vordefinierten Bewegungen dieses Abschnitts dargestellt werden.

2. Steuereinheit (150) nach Anspruch 1, wobei die Anweisungen, die Steuereinheit (150) weiter zu Folgendem veranlassen:
Steuern der Robotervorrichtung (160) basierend auf der Abfolge oder einer Auswahl einer angezeigten vordefinierten Bewegung der Abfolge durch einen Benutzer, um die interventionelle Vorrichtung (101) über mindestens einen Abschnitt des durch die Abfolge oder Auswahl bestimmten Pfads einzuführen.

3. Steuereinheit (150) nach Anspruch 1 oder 2, wobei die Anweisungen die Steuereinheit (150) weiter zu Folgendem veranlassen:
Anzeigen (180) mindestens eines Abschnitts des Pfads auf einer Anzeigeeinrichtung (180), so dass die vordefinierten Bewegungen dieses Abschnitts entsprechend ihrer Reihenfolge in der Abfolge dargestellt werden.

4. Steuereinheit nach einem der vorstehenden Ansprüche, wobei die Anzeigeeinheit (180) zukünftige
erwartete oder empfohlene vordefinierte Bewegungen innerhalb der Abfolge umfasst.

5. Steuereinheit nach Anspruch 4, wobei die Anzeigeeinheit (180) weiter aktuelle und vergangene vordefinierte Bewegungen innerhalb der Abfolge umfasst.

6. Steuereinheit nach Anspruch 5, wobei die Anweisungen die Steuereinheit (150) weiter veranlassen,
die vordefinierte Bewegung hervorzuheben, die derzeit aktiv ist.

7. Steuereinheit (150) nach einem von Anspruch 1 bis 6, wobei die Anweisungen die Steuereinheit (150) weiter zu Folgendem veranlassen:
Bestimmen, basierend auf Analysieren der Bilddaten, einer Vielzahl von zukünftigen empfohlenen Pfaden für die interventionelle Vorrichtung (101) durch die anatomische Struktur unter Verwendung der im Speicher (151) gespeicherten Module vordefinierter Bewegungen, wobei jeder zukünftige empfohlene Pfad durch eine Abfolge vordefinierter Bewegungen definiert ist; und
Anzeigen (180) von Darstellungen mindestens von Abschnitten der Vielzahl zukünftiger empfohlener Pfade, wobei jede Darstellung eines Pfades vordefinierte Bewegungen mindestens eines Teils der entsprechenden Abfolge umfasst.

8. Steuereinheit nach Anspruch 7, wobei die Anweisungen die Steuereinheit (150) weiter veranlassen, dem Benutzer eine Möglichkeit zu geben, einen empfohlenen Pfad oder vordefinierte Bewegungen zu ändern und/oder zu akzeptieren.

9. Steuereinheit (150) nach einem der vorstehenden Ansprüche, wobei die vordefinierten Bewegungen
mindestens eines beinhalten von:
Ausrichten des distalen Abschnitts der äußeren Vorrichtung mit dem distalen Abschnitt der inneren Vorrichtung, um übereinstimmende distale Abschnitte der interventionellen Vorrichtung (101) bereitzustellen;
Drehen ausgerichteter, übereinstimmender distaler Abschnitte der interventionellen Vorrichtung (101), um in eine Richtung zum Zielast zu zeigen; und
Zurückziehen der ausgerichteten, übereinstimmenden distalen Abschnitte der interventionellen Vorrichtung (101) zu einem Zielpunkt in einer Bildebene des Bildes, wobei der Zielpunkt eine bekannte Beziehung relativ zum Zielast hat.

10. Steuereinheit (150) nach Anspruch 9, wobei das Ausrichten des distalen Abschnitts der äußeren Vorrichtung mit dem distalen Abschnitt der inneren Vorrichtung das Zurückziehen der inneren Vorrichtung in die äußere Vorrichtung umfasst, wobei die Anweisungen die Steuereinheit (150) weiter veranlassen, das Zurückziehen der inneren Vorrichtung in die äußere Vorrichtung mit der entsprechenden vordefinierten Bewegung umzusetzen, umfassend:
Bestimmen einer freiliegenden Länge der inneren Vorrichtung in Pixeln, die sich vom distalen Abschnitt der äußeren Vorrichtung erstreckt;
Bestimmen einer Fehlermetrik basierend auf der freiliegenden Länge der inneren Vorrichtung und
Zurückziehen der inneren Vorrichtung relativ zu der äußeren Vorrichtung unter Verwendung der Fehlermetrik in einer Proportional-Integral-Differential-, (PID)-, Regelschleife, wodurch die freiliegende Länge der inneren Vorrichtung verringert wird, bis ein Toleranzfehler eingehalten wird.

11. Steuereinheit (150) nach Anspruch 9, wobei die Anweisungen die Steuereinheit (150) weiter veranlassen, das Zurückziehen der ausgerichteten, übereinstimmenden distalen Abschnitte der interventionellen Vorrichtung (101) zum Zielpunkt der entsprechenden vordefinierten Bewegung umzusetzen, umfassend:
Empfangen einer Auswahl einer Stelle des Zielpunkts im Bild des Abschnitts der interventionellen Vorrichtung (101) und der verzweigten Kreuzung;
Messen einer Länge in Pixeln der interventionellen Vorrichtung (101), die über die Stelle des Zielpunkts hinausgeht;
anfängliches Zurückziehen der interventionellen Vorrichtung (101) durch Verkürzen der Länge in Pixeln mit einer ersten Geschwindigkeit, wenn die Länge in Pixeln eine vorgegebene Distanz überschreitet; und
weiter Zurückziehen der interventionellen Vorrichtung (101) durch Verkürzen der Länge in Pixeln mit einer zweiten Geschwindigkeit, wenn die Länge in Pixeln die vorgegebene Distanz nicht mehr überschreitet, wobei die zweite Geschwindigkeit langsamer ist als die erste Geschwindigkeit.

12. Steuereinheit (150) nach einem der vorstehenden Ansprüche, wobei die Anweisungen die Steuereinheit (150) weiter zu Folgendem veranlassen:
getrenntes Drehen der inneren Vorrichtung und der äußeren Vorrichtung, um Krümmungen der inneren Vorrichtung und der äußeren Vorrichtung auszurichten.

13. Steuereinheit (150) nach Anspruch 12, wobei die Anweisungen die Steuereinheit (150) weiter zu Folgendem veranlassen:
Identifizieren einer äußeren Tangente zu einer Kurve der äußeren Vorrichtung als Referenzachse;
Identifizieren einer inneren Tangente zu einer Kurve der inneren Vorrichtung;
Bestimmen eines Winkels zwischen einer inneren Tangente der inneren Vorrichtung und der Referenzachse;
getrenntes Drehen der inneren Vorrichtung und der äußeren Vorrichtung, um Krümmungen der inneren Vorrichtung und der äußeren Vorrichtung auszurichten, und, wenn der Winkel 180 Grad überschreitet, Drehen der inneren Vorrichtung in Richtung der Referenzachse, bis der Winkel zwischen der inneren Tangente der inneren Vorrichtung und der Referenzachse weniger als 180 Grad beträgt.

14. Steuereinheit (150) nach Anspruch 12 oder 13, wobei die Anweisungen die Steuereinheit (150) veranlassen, die getrennte Drehung der inneren Vorrichtung und der äußeren Vorrichtung umzusetzen, um Krümmungen der inneren Vorrichtung und der äußeren Vorrichtung auszurichten, und die Steuereinheit (150) weiter zu Folgendem zu veranlassen:
wenn die Kurve der äußeren Vorrichtung zunächst nicht im Bild sichtbar ist, Ändern einer Perspektive der Bildgebungsvorrichtung oder Drehen der äußeren Vorrichtung, bis die Kurve im Bild sichtbar ist.

15. System (100) zum Steuern einer Robotervorrichtung (160), die dazu konfiguriert ist, eine interventionelle Vorrichtung (101) in eine anatomische Struktur eines Subjekts bis zu einem Ziel einzuführen, wobei die interventionelle Vorrichtung (101) eine äußere Vorrichtung und eine innere Vorrichtung umfasst, die beweglich innerhalb der äußeren Vorrichtung positioniert ist, wobei das System (100) Folgendes umfasst:
eine Bildgebungsschnittstelle, die dazu konfiguriert ist, Bilddaten, die einem Bild entsprechen, von einer Bildgebungsvorrichtung zu empfangen, wobei das Bild einen Abschnitt der interventionellen Vorrichtung (101) und eine verzweigte Schnittstelle einer Vielzahl von Ästen der anatomischen Struktur beinhaltet, einschließlich eines Hauptasts und eines Zielasts, der vom Hauptast abzweigt;
eine Benutzerschnittstelle, die zum Empfangen von Eingaben von einem Benutzer konfiguriert ist;
eine Schnittstelle, die dazu angeordnet ist, das System (100) mit einer Robotervorrichtung (160) zu verbinden, um die interventionelle Vorrichtung (101) in die anatomische Struktur des Subjekts einzuführen;
eine Steuereinheit (150) für eine Robotervorrichtung (160) nach einem der Ansprüche 1 bis 14, wobei die Steuereinheit (150) für die Robotervorrichtung (160) weiter dazu konfiguriert ist, basierend auf der Abfolge oder einer Auswahl einer angezeigten vordefinierten Bewegung durch einen Benutzer die Robotervorrichtung (160) zu steuern, um die interventionelle Vorrichtung (101) über mindestens einen Abschnitt des durch die Abfolge oder Auswahl bestimmten Pfads einzuführen; und
eine Anzeigeeinheit (180), die dazu konfiguriert ist, mindestens die Verzweigungsschnittstelle der Vielzahl von Verzweigungen der anatomischen Struktur, den distalen Abschnitt der äußeren Vorrichtung und den distalen Abschnitt der inneren Vorrichtung der interventionellen Vorrichtung (101) anzuzeigen (180), während die Steuereinheit (150) der Robotervorrichtung (160) die Robotervorrichtung (160) steuert.

## Revendications

1. Dispositif de commande (150) pour commander un dispositif robotique (160) configuré pour manœuvrer un
dispositif d'intervention (101) dans une structure anatomique d'un sujet, le dispositif d'intervention (101) comprenant un dispositif externe et un dispositif interne positionné de manière mobile à l'intérieur du dispositif externe, le dispositif de commande (150) comprenant :
une mémoire (151) qui stocke des instructions et une bibliothèque d'une pluralité de modules de mouvements prédéfinis pour naviguer à travers des structures anatomiques ; et
un processeur (1010) qui exécute les instructions, dans lequel, lorsqu'elles sont exécutées par le processeur (1010), les instructions amènent le dispositif de commande (150) à :
recevoir, d'un dispositif d'imagerie, des données d'image provenant d'une image d'une partie du dispositif d'intervention (101) et d'une intersection ramifiée d'une pluralité de branches de la structure anatomique, incluant une branche principale et une branche cible qui est ramifiée à partir de la branche principale ;
analyser les données d'image pour mesurer au moins l'un d'un emplacement ou d'une orientation d'une partie distale du dispositif externe et d'une partie distale du dispositif interne dans l'image, par rapport à un système vasculaire incluant la branche principale et la branche cible et une intersection de la branche principale et de la branche cible dans l'image ;
déterminer, sur la base de l'analyse des données d'image, un trajet pour le dispositif d'intervention (101) à travers la structure anatomique jusqu'à une cible à l'aide des modules de mouvements prédéfinis stockés dans la mémoire (151), le trajet étant défini par une séquence de mouvements prédéfinis ; et
afficher (180) au moins une partie du trajet sur un écran (180) de telle sorte que les mouvements prédéfinis de cette partie soient représentés.

2. Dispositif de commande (150) selon la revendication 1, dans lequel les instructions amènent en outre le dispositif de commande (150) à :
commander, sur la base de la séquence ou d'une sélection par un utilisateur d'un mouvement prédéfini affiché de la séquence, le dispositif robotique (160) pour manœuvrer le dispositif d'intervention (101) sur au moins une partie du trajet déterminé par la séquence ou la sélection.

3. Dispositif de commande (150) selon la revendication 1 ou 2, dans lequel les instructions amènent en outre le
dispositif de commande (150) à :
afficher (180) au moins une partie du trajet sur un écran (180) de telle sorte que les mouvements prédéfinis de cette partie soient représentés selon leur ordre dans la séquence.

4. Dispositif de commande selon l'une quelconque des revendications précédentes, dans lequel l'écran (180) comprend
des futurs mouvements prédéfinis attendus ou recommandés dans la séquence.

5. Dispositif de commande selon la revendication 4, dans lequel l'écran (180) comprend en outre
des mouvements prédéfinis actuels et passés dans la séquence.

6. Dispositif de commande selon la revendication 5, dans lequel les instructions amènent en outre le dispositif de commande (150)
à mettre en évidence le mouvement prédéfini qui est actuellement actif.

7. Dispositif de commande (150) selon l'une quelconque des revendications 1 à 6, dans lequel les instructions amènent en outre le dispositif de commande (150) à :
déterminer, sur la base de l'analyse des données d'image, une pluralité de futurs trajets recommandés pour le dispositif d'intervention (101) à travers la structure anatomique à l'aide des modules de mouvements prédéfinis stockés dans la mémoire (151), chaque futur trajet recommandé étant défini par une séquence de mouvements prédéfinis ; et
afficher (180) des représentations d'au moins des parties de la pluralité de futurs trajets recommandés, chaque représentation d'un trajet comprenant des mouvements prédéfinis d'au moins une partie de la séquence correspondante.

8. Dispositif de commande selon la revendication 7, dans lequel les instructions amènent en outre le dispositif de commande (150) à fournir une option à l'utilisateur pour modifier et/ou accepter un trajet recommandé ou des mouvements prédéfinis.

9. Dispositif de commande (150) selon l'une quelconque des revendications précédentes, dans lequel les mouvements prédéfinis
incluent au moins l'un de :
l'alignement de la partie distale du dispositif externe avec la partie distale du dispositif interne pour fournir des parties distales coïncidentes du dispositif d'intervention (101) ;
la rotation de parties distales coïncidentes alignées du dispositif d'intervention (101) pour les faire pointer dans une direction vers la branche cible ; et
la rétraction des parties distales coïncidentes alignées du dispositif d'intervention (101) vers un point cible dans un plan d'image de l'image, dans lequel le point cible présente une relation connue par rapport à la branche cible.

10. Dispositif de commande (150) selon la revendication 9, dans lequel l'alignement de la partie distale du dispositif externe avec la partie distale du dispositif interne comprend la rétraction du dispositif interne dans le dispositif externe, dans lequel les instructions amènent en outre le dispositif de commande (150) à mettre en œuvre ladite rétraction du dispositif interne dans le dispositif externe du mouvement prédéfini correspondant, comprenant :
la détermination d'une longueur exposée en pixels du dispositif interne s'étendant à partir de la partie distale du dispositif externe ;
la détermination d'une mesure d'erreur basée sur la longueur exposée du dispositif interne, et
la rétraction du dispositif interne par rapport au dispositif externe à l'aide de la mesure d'erreur dans une boucle de commande proportionnelle-intégrale-dérivée (PID), ce qui permet de diminuer la longueur exposée du dispositif interne jusqu'à ce qu'une erreur de tolérance soit satisfaite.

11. Dispositif de commande (150) selon la revendication 9, dans lequel les instructions amènent en outre le dispositif de commande (150) à mettre en œuvre ladite rétraction des parties distales coïncidentes alignées du dispositif d'intervention (101) jusqu'au point cible du mouvement prédéfini correspondant, comprenant :
la réception d'une sélection d'un emplacement du point cible dans l'image de la partie du dispositif d'intervention (101) et de l'intersection ramifiée ;
la mesure d'une longueur en pixels du dispositif d'intervention (101) s'étendant au-delà de l'emplacement du point cible ;
la rétraction initiale du dispositif d'intervention (101) en raccourcissant la longueur en pixels à une première vitesse lorsque la longueur en pixels dépasse une distance prédéterminée ; et
la poursuite de la rétraction du dispositif d'intervention (101) en raccourcissant la longueur en pixels à une seconde vitesse lorsque la longueur en pixels ne dépasse plus la distance prédéterminée, dans lequel la seconde vitesse est plus lente que la première vitesse.

12. Dispositif de commande (150) selon l'une quelconque des revendications précédentes, dans lequel les instructions amènent en outre le dispositif de commande (150) à :
faire pivoter séparément le dispositif interne et le dispositif externe pour aligner des courbures du dispositif interne et du dispositif externe.

13. Dispositif de commande (150) selon la revendication 12, dans lequel les instructions amènent en outre le dispositif de commande (150) à :
identifier une tangente externe à une courbe du dispositif externe comme axe de référence ;
identifier une tangente interne à une courbe du dispositif interne ;
déterminer un angle entre une tangente interne du dispositif interne et l'axe de référence ;
faire tourner séparément le dispositif interne et le dispositif externe pour aligner des courbures du dispositif interne et du dispositif externe et, lorsque l'angle dépasse 180 degrés, faire tourner le dispositif interne vers l'axe de référence jusqu'à ce que l'angle entre la tangente interne du dispositif interne et l'axe de référence soit inférieur à 180 degrés.

14. Dispositif de commande (150) selon la revendication 12 ou 13, dans lequel les instructions amenant le dispositif de commande (150) à mettre en œuvre ladite rotation séparée du dispositif interne et du dispositif externe pour aligner des courbures du dispositif interne et du dispositif externe, amènent en outre le dispositif de commande (150) à :
lorsque la courbe du dispositif externe n'est initialement pas visible dans l'image, changer une perspective du dispositif d'imagerie ou faire pivoter le dispositif externe jusqu'à ce que la courbe soit visible dans l'image.

15. Système (100) pour commander un dispositif robotique (160) configuré pour manœuvrer un dispositif d'intervention (101) dans une structure anatomique d'un sujet vers une cible, le dispositif d'intervention (101) comprenant un dispositif externe et un dispositif interne positionné de manière mobile à l'intérieur du dispositif externe, le système (100) comprenant :
une interface d'imagerie configurée pour recevoir des données d'image correspondant à une image provenant d'un dispositif d'imagerie, l'image incluant une partie du dispositif d'intervention (101) et une intersection ramifiée d'une pluralité de branches de la structure anatomique, incluant une branche principale et une branche cible qui est ramifiée à partir de la branche principale ;
une interface utilisateur configurée pour recevoir une entrée d'un utilisateur ;
une interface agencée pour connecter le système (100) à un dispositif robotique (160) pour manœuvrer le dispositif d'intervention (101) dans la structure anatomique du sujet ;
un dispositif de commande (150) de dispositif robotique (160) selon l'une quelconque des revendications 1 à 14, le dispositif de commande (150) de dispositif robotique (160) étant en outre configuré pour commander, sur la base de la séquence ou d'une sélection par un utilisateur d'un mouvement prédéfini affiché, le dispositif robotique (160) pour manœuvrer le dispositif d'intervention (101) sur au moins une partie du trajet déterminé par la séquence ou la sélection ; et
un écran (180) configuré pour afficher (180) au moins l'intersection de branches de la pluralité de branches de la structure anatomique, la partie distale du dispositif externe et la partie distale du dispositif interne du dispositif d'intervention (101), en même temps que le dispositif de commande (150) de dispositif robotique (160) commande le dispositif robotique (160).
